# EUROPÄISCHE PATENTSCHRIFT

(11) **EP 1 095 015 B1**
(45) Veröffentlichungstag und Bekanntmachung des Hinweises auf die Patenterteilung: **01.06.2005**
(21) Anmeldenummer: 99936460.7
(22) Anmeldetag: 25.06.1999
(51) Int. Cl.: C07C 311/21

(54) **SULFONYLAMINO-CARBONSÄURE-N-ARYLAMIDE ALS GUANYLATCYCLASE-AKTIVATOREN**
SULFONYLAMINO CARBOXYLIC ACID N-ARYLAMIDES AS GUANYLATE CYCLASE ACTIVATORS
N-ARYLAMIDES D'ACIDE SULFONYLAMINOCARBOXYLIQUE UTILISES COMME ACTIVATEURS DE LA GUANYLATE CYCLASE

(30) Priorität: 08.07.1998 DE 19830431
(43) Veröffentlichungstag der Anmeldung: 02.05.2001
(73) Patentinhaber: Aventis Pharma Deutschland GmbH, 65929 Frankfurt am Main (DE)
(72) Erfinder: SCHINDLER, Ursula, D-65812 Bad Soden (DE); SCHÖNAFINGER, Karl, D-63755 Alzenau (DE); STROBEL, Hartmut, D-65835 Liederbach (DE)
(86) Internationale Anmeldenummer: PCT/EP1999/004427
(87) Internationale Veröffentlichungsnummer: WO 2000/002850

(56) Entgegenhaltungen:
- EP-A- 0 347 168
- EP-A- 0 420 804
- EP-A- 0 420 805
- EP-A- 0 947 500
- WO-A-98/27053
- DE-A- 3 523 705
- FR-A- 1 579 473
- GB-A- 865 735
- DATABASE CHEMABS [Online] CHEMICAL ABSTRACTS SERVICE, COLUMBUS, OHIO, US STN, CAPLUS accession no. 1997:265454, XP002130171 -& CHEMICAL ABSTRACTS, vol. 126, no. 21, 26. Mai 1997 (1997-05-26) Columbus, Ohio, US; abstract no. 277494d, XP002130160 & JP 09 059236 A (DAIICHI SEIYAKU CO) 4. März 1997 (1997-03-04)
- DATABASE CHEMABS [Online] CHEMICAL ABSTRACTS SERVICE, COLUMBUS, OHIO, US STN, CAPLUS accession no. 1984:209423, XP002130172 -& CHEMICAL ABSTRACTS, vol. 100, no. 25, 18. Juni 1984 (1984-06-18) Columbus, Ohio, US; abstract no. 209423t, XP002130161 & JP 59 016871 A (SHIONOGI AND CO LTD) 28. Januar 1984 (1984-01-28)
- DATABASE CHEMABS [Online] CHEMICAL ABSTRACTS SERVICE, COLUMBUS, OHIO, US STN, CAPLUS accession no. 1985:70111, XP002130173 -& CHEMICAL ABSTRACTS, vol. 102, no. 8, 25. Februar 1985 (1985-02-25) Columbus, Ohio, US; abstract no. 70111k, XP002130162 & JP 59 162547 A (KONISHIROKU PHOTO INDUSTRY CO LTD) 13. September 1984 (1984-09-13)
- DATABASE CHEMABS [Online] CHEMICAL ABSTRACTS SERVICE, COLUMBUS, OHIO, US STN, CAPLUS accession no. 1969:77907, XP002130174 & B.M. BOLOTIN ET AL: ZH. VSES. KHIM. OBSHCHEST., Bd. 13, Nr. 4, 1968, Seiten 475-476,
- DATABASE CHEMABS [Online] CHEMICAL ABSTRACTS SERVICE, COLUMBUS, OHIO, US STN, CAPLUS accession no. 1992:439809, XP002130175 & F.S. MIKHAILITSYN ET AL: MED. PARAZITOL. PARAZIT. BOLEZNI, Nr. 6, 1991, Seiten 52-53,
- DATABASE CHEMABS [Online] CHEMICAL ABSTRACTS SERVICE, COLUMBUS, OHIO, US STN, CAPLUS accession no. 1986:33896, XP002130176 -& CHEMICAL ABSTRACTS, vol. 104, no. 5, 3. Februar 1986 (1986-02-03) Columbus, Ohio, US; abstract no. 33896r, XP002130163 in der Anmeldung erwähnt & RO 79 282 B (INSTITUTUL DE CERCETARI CHIMICO-FARMACEUTICE) 17. August 1982 (1982-08-17)
- DATABASE CHEMABS [Online] CHEMICAL ABSTRACTS SERVICE, COLUMBUS, OHIO, US STN, CAPLUS accession no. 1993:580796, XP002130177 -& CHEMICAL ABSTRACTS, vol. 119, no. 17, 25. Oktober 1993 (1993-10-25) Columbus, Ohio, US; abstract no. 180796w, XP002130164 & SU 1 766 919 A (F.S. MIKHAJLITSYN ET AL) 7. Oktober 1992 (1992-10-07)
- DATABASE CHEMABS [Online] CHEMICAL ABSTRACTS SERVICE, COLUMBUS, OHIO, US STN, CAPLUS accession no. 1973:418616, XP002130178 -& CHEMICAL ABSTRACTS, vol. 79, no. 3, 23. Juli 1973 (1973-07-23) Columbus, Ohio, US; abstract no. 18616w, XP002130165 & N.I. CHERNOVA ET AL: KHIM. GETEROSIKL. SOEDIN., Nr. 4, 1973, Seiten 472-478,
- DATABASE CHEMABS [Online] CHEMICAL ABSTRACTS SERVICE, COLUMBUS, OHIO, US STN, CAPLUS accession no. 1971:99956, XP002130179 -& CHEMICAL ABSTRACTS, vol. 74, no. 19, 10. Mai 1971 (1971-05-10) Columbus, Ohio, US; abstract no. 99956d, XP002130166 & M.V. LOSEVA ET AL: KHIM. GETEROTSIKL. SOEDIN., Nr. 12, 1970, Seiten 1597-1601,
- DATABASE CHEMABS [Online] CHEMICAL ABSTRACTS SERVICE, COLUMBUS, OHIO, US STN, CAPLUS accession no.1972:33569, XP002130180 & M.V. LOSEVA ET AL: KHIM. GETEROTSIKL. SOEDIN., Bd. 7, Nr. 8, 1971, Seiten 1028-1032,
- DATABASE CHEMABS [Online] CHEMICAL ABSTRACTS SERVICE, COLUMBUS, OHIO, US STN, CAPLUS accession no. 1972:526528, XP002130181 & M.V. LOSEVA ET AL: KHIM. GETEROTSIKL. SOEDIN., Nr. 5, 1972, Seiten 616-621,
- DATABASE CHEMABS [Online] CHEMICAL ABSTRACTS SERVICE, COLUMBUS, OHIO, US STN, CAPLUS accession no. 1972:564616, XP002130182 -& CHEMICAL ABSTRACTS, vol. 77, no. 25, 18. Dezember 1972 (1972-12-18) Columbus, Ohio, US; abstract no. 164616w, XP002130167 & B.M. BOLOTIN ET AL: TR. VSES. NAUCH.-ISSLED. INST. KHIM. REAKTIV. OSOBO CHIST. KHIM. VESHCHESTV, Nr. 33, 1971, Seiten 104-118,
- DATABASE CHEMABS [Online] CHEMICAL ABSTRACTS SERVICE, COLUMBUS, OHIO, US STN, CAPLUS accession no. 1974:491449, XP002130183 -& CHEMICAL ABSTRACTS, vol. 81, no. 15, 14. Oktober 1974 (1974-10-14) Columbus, Ohio, US; abstract no. 91449q, XP002130168 & B.M. BOLOTIN ET AL: KHIM. GETEROTSIKL. SOEDIN., Nr. 6, 1974, Seiten 760-763,
- DATABASE CHEMABS [Online] CHEMICAL ABSTRACTS SERVICE, COLUMBUS, OHIO, US STN, CAPLUS accession no. 1983:557667, XP002130184 -& CHEMICAL ABSTRACTS, vol. 99, no. 19, 7. November 1983 (1983-11-07) Columbus, Ohio, US; abstract no. 157667q, XP002130169 & L.V. CHURSINOVA ET AL: IZV. TIMIRYAZESK. S-KH. AKAD., Nr. 4, 1983, Seiten 170-172,
- DATABASE CHEMABS [Online] CHEMICAL ABSTRACTS SERVICE, COLUMBUS, OHIO, US STN, CAPLUS accession no. 1987:138322, XP002130185 -& CHEMICAL ABSTRACTS, vol. 106, no. 17, 27. April 1987 (1987-04-27) Columbus, Ohio, US; abstract no. 138322v, XP002130170 & H. BRAEUNIGER ET AL: WISS. Z. WILHELM-PIECK-UNIV. ROSTOCK, NATURWISS. REIHE, Bd. 33, Nr. 8, 1984, Seiten 53-61,
- Y. TOMINAGA ET AL: J. HETEROCYCL. CHEM., Bd. 27, Nr. 5, 1990, Seiten 1217-1225, XP000867501
- H.G. MCFADDEN ET AL: AUST. J. CHEM., Bd. 46, Nr. 6, 1993, Seiten 873-886, XP000196744
- L. GERA ET AL: ACTA CHIM. ACAD. SCI. HUNG., Bd. 99, Nr. 2, 1979, Seiten 175-192, XP002130159
- A.M. EL-NAGGAR ET AL: POL. J. CHEM., Bd. 56, Nr. 10-12, 1982, Seiten 1279-1285, XP000874826

## Beschreibung

Gegenstand der vorliegenden Erfindung sind Verbindungen der Formel I, 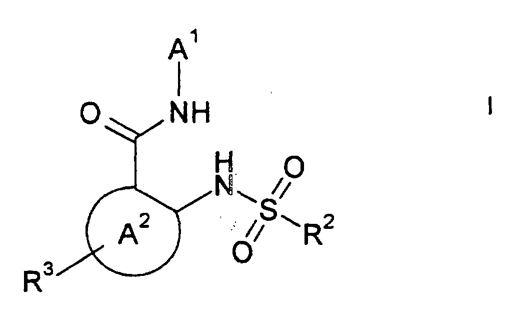 in der A¹, A², R² und R³ die unten angegebenen Bedeutungen haben, die wertvolle Arzneimittelwirkstoffe für die Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft die Verwendung von Verbindungen der Formel I zur Therapie und Prophylaxe der bezeichneten Krankheitszustände und zur Herstellung von Arzneimitteln dafür, neue Verbindungen der Formel I, sie enthaltende pharmazeutische Präparate und Verfahren zu ihrer Herstellung.

cGMP ist ein wichtiger intrazellulärer Botenstoff, der über die Modulation cGMPabhängiger Proteinkinasen, Phosphodiesterasen und lonenkanäle eine Reihe von pharmakologischen Effekten auslöst. Beispiele sind die Glattmuskelrelaxation, die Inhibition der Thrombozytenaktivierung und die Hemmung von Glattmuskelzellproliferation und Leukozytenadhäsion. cGMP wird durch partikuläre und lösliche Guanylatcyclasen als Antwort auf eine Reihe extrazellulärer und intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer α- und einer β-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen α₁ und β₁ werden hauptsächlich in Gehirn und Lunge exprimiert, während β₂ vor allem in Leber und Niere gefunden wird. In humanem fötalen Gehim konnte der Subtyp α₂ nachgewiesen werden. Die als α₃ und β₃ bezeichneten Untereinheiten wurden aus menschlichem Gehim isoliert und sind homolog zu α₁ und β₁. Neuere Arbeiten weisen auf eine α₂ᵢ-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über β₁-Cys-78 und/oder β₁-His-105 gebunden ist und Teil des regulatorischen Zentrums ist.

Unter pathologischen Bedingungen kann die Bildung Guanylatcyclase-aktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMPvermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung bzw. Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Vesely in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Vesely, Eur. J Clin. Invest. 15 (1985) 258; D. L. Vesely, Biochem. Biophys. Res. Comm. 88 (1979) 1244). Eine Stimulation Häm-freier Guanylatcyclase durch Protoporphyrin IX wurde durch lgnarro et al. (Adv. Pharmacol. 26 (1994) 35) nachgewiesen. Pettibone et al. (Eur. J. Pharmacol. 116 (1985) 307) beschrieben für Diphenyliodoniumhexafluorophoshat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Wu et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung.

Eine Reihe von Sulfonylamino-carbonsäure-N-arylamiden der Formel I sind bereits bekannt. Verbindungen dieses Typs finden zum Beispiel Verwendung bei der Herstellung von photographischen Materialien (siehe zum Beispiel Chemical Abstracts 119, 105 755; 116, 245 151 und 104, 177 628) und enthalten für diesen Zweck in der N-Arylgruppe dann im allgemeinen als Substituenten leicht oxidierbare Gruppen wie zum Beispiel zwei zueinander in para-Stellung stehende Hydroxygruppen. Für verschiedene Verbindungen der Formel I ist auch bereits eine pharmakologische Wirkung beschrieben. So wird zum Beispiel in der DE-A-35 23 705 für eine Reihe von 2-Phenylsulfonylamino-benzoesäureamiden eine anthelminthische Wirkung beschrieben. Antiparasitäre, antimikrobielle oder fungizide Wirkungen von 2-Sulfonylamino-benzoesäure-N-(hetero)aryl-amiden werden zum Beispiel auch in der EP-A-420 805 und in Chemical Abstracts 122, 136 749; 120, 560; 119, 116 978; 116, 228 237; 116, 207 806; 115, 158 666 und 106, 152 850 erwähnt. Gemäß der EP-A-347 168 sind bestimmte Verbindungen der Formel I mit einer Pivalinsäurephenylesterstruktur Elastase-Inhibitoren und können bei der Behandlung der Atherosklerose oder der Arthritis Verwendung finden. In Chemical Abstracts 104, 33 896 wird für bestimmte 2-Sulfonylamino-benzoesäure-N-phenoxyphenyl-amide eine psychotrope Wirkung beschrieben. Verschiedene 2-Trifluormethylsulfonylamino- und 2-Methylsulfonylamino-substituierte Benzoesäureamide werden in Hypertension 15 (1998) 823, J. Med. Chem. 33 (1990) 1312, EP-A-253 310, EP-A-324 377, EP-A-449 699, EP-A-530 702 und US-A-4 880 804 als antihypertensiv wirksame Angiotensin-II-Rezeptorantagonisten beschrieben.

Überraschend wurde nun gefunden, daß die Verbindungen der Formel I eine starke Guanylatcyclase-Aktivierung bewirken, aufgrund derer sie zur Therapie und Prophylaxe von Krankheiten geeignet sind, die mit einem niedrigen cGMP-Spiegel verbunden sind.

Die vorliegende Erfindung betrifft somit die Verwendung von Verbindungen der Formel I, 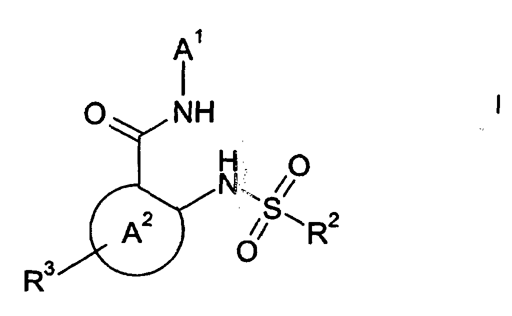 in der
A¹ für einen Rest aus der Reihe Phenyl und Naphthyl steht, die alle durch einen oder mehrere gleiche oder verschiedene Reste R¹ substituiert sein können;
der Ring A², der die Kohlenstoffatome umfaßt, die die Gruppen CO-NH- und NH-SO₂R² tragen, für einen Benzolring steht;
R¹ für Halogen, Aryl, CF₃, NO₂, OH, -O-(C₁-C₇)-Alkyl, -O-(C₂-C₄)-Alkyl-O-(C₁-C₇)-Alkyl, -O-Aryl, (C₁-C₂)-Alkylendioxy, NR⁵R⁶, CN, CO-NR⁵R⁶, COOH, CO-O-(C₁-C₅)-Alkyl, Heterocyclyl, CHO, CO-(C₁-C₁₀)-Alkyl, co-Aryl oder für (C₁-C₁₀)-Alkyl, das durch einen oder mehrere gleiche oder verschiedene Reste R⁴ substituiert sein kann, steht;
R² für Phenyl, das durch einen oder mehrere gleiche oder verschiedene Substituenten aus der Reihe Halogen, CF₃, (C₁-C₃)-Alkyl, Cyan, Nitro und (C₁-C₃)-Alkoxy substituiert sein kann, steht;
R³ für einen oder mehrere gleiche oder verschiedene Substituenten aus der Reihe Wasserstoff und Halogen steht;
R⁴ für Fluor, OH, -O-(C₁-C₁₀)-Alkyl, -O-(C₁-C₇)-Alkyl-R⁷, -O-Aryl, SH, -S-(C₁-C₁₀)-Alkyl, -S-(C₁-C₇)-Alkyl-R⁷, -S-Aryl, -P(O)(O-(C₁-C₅)-Alkyl)₂, -P(O)(OH)₂, CN, NR⁸R⁹, CO-NH₂, CO-NH-(C₁-C₃)-Alkyl, CO-N((C₁-C₃)-Alkyl)₂, COOH, CO-O-(C₁-C₅)-Alkyl, Heterocyclyl oder Oxo steht;
R⁵ für Wasserstoff, (C₁-C₁₀)-Alkyl, das durch einen oder mehrere gleiche oder verschiedene Substituenten R⁴ und/oder durch Aryl substituiert sein kann, Aryl, Heterocyclyl, CO-(C₁-C₁₀)-Alkyl, CO-Aryl, CO-Heterocyclyl, SO₂-(C₁-C₁₀)-Alkyl, SO₂-Aryl oder SO₂-Heterocyclyl steht;
R⁶ unabhängig von R⁵ eine der für R⁵ angegebenen Bedeutungen hat, oder R⁵ und R⁶ zusammen mit dem Stickstoffatom, an das sie gebunden sind, einen 5-gliedrigen bis 8-gliedrigen gesättigten oder teilweise ungesättigten Ring bilden, der zusätzlich zu dem die Gruppen R⁵ und R⁶ tragenden Stickstoffatom noch ein oder mehrere weitere Ring-Heteroatome aus der Reihe N, O und S enthalten kann und der durch einen oder mehrere gleiche oder verschiedene Substituenten aus der Reihe Fluor, (C₁-C₅)-Alkyl, (C₁-C₃)-Hydroxyalkyl, -(C₁-C₃)-Alkyl-O-(C₁-C₄)-Alkyl, Aryl, CF₃, OH, -O-(C₁-C₇)-Alkyl, -O-Aryl, -O-(C₂-C₄)-Alkyl-O-(C₁-C₇)-Alkyl, (C₂-C₃)-Alkylendioxy, NR⁸R⁹ CN, CO-NH₂, CO-NH-(C₁-C₃)-Alkyl, CO-N((C₁-C₃)-Alkyl)₂, COOH, CO-O-(C₁-C₅)-Alkyl, CHO, CO-(C₁-C₅)-Alkyl, S(O)ₙ-(C₁-C₄)-Alkyl, S(O)ₙ-NH₂, S(O)ₙ-NH-(C₁-C₃)-Alkyl, S(O)ₙ-N((C₁-C₃)-Alkyl)₂, Oxo, -(CH₂)ₘ-NH₂, -(CH₂)ₘ-NH-(C₁-C₄)-Alkyl und -(CH₂)ₘ-N((C₁-C₄)-Alkyl)₂ substituiert sein kann, wobei in dem Substituenten -(CH₂)ₘ-N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁷ für OH, -O-(C₁-C₇)-Alkyl, NH₂, -NH-(C₁-C₄)-Alkyl oder -N((C₁-C₄)-Alkyl)₂ steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁸ für Wasserstoff oder (C₁-C₇)-Alkyl, das durch einen oder mehrere gleiche oder verschiedene Substituenten aus der Reihe OH, -O-(C₁-C₅)-Alkyl, NH₂, -NH-(C₁-C₄)-Alkyl und -N((C₁-C₄)-Alkyl)₂ substituiert sein kann, steht;
R⁹ unabhängig von R⁸ eine der Bedeutungen von R⁸ hat oder für CO-(C₁-C₄)-Alkyl steht;
Aryl für Phenyl, Naphthyl oder Heteroaryl steht, die alle durch einen oder mehrere gleiche oder verschiedene Substituenten aus der Reihe Halogen, (C₁-C₅)-Alkyl, Phenyl, Tolyl, CF₃, NO₂, OH, -O-(C₁-C₅)-Alkyl, -O-(C₂-C₄)-Alkyl-O-(C₁-C₃)-Alkyl, (C₁-C₂)-Alkylendioxy, NH₂, -NH-(C₁-C₃)-Alkyl, -N((C₁-C₃)-Alkyl)₂, NH-CHO, -NH-CO-(C₁-C₅)-Alkyl, CN, CO-NH₂, CO-NH-(C₁-C₃)-Alkyl, CO-N((C₁-C₃)-Alkyl)₂, COOH, CO-O-(C₁-C₅)-Alkyl, Heterocyclyl, CHO, CO-(C₁-C₅)-Alkyl, S(O)ₙ-(C₁-C₄)-Alkyl, S(O)ₙ-Phenyl, S(O)ₙ-Tolyl, S(O)₂-NH₂, S(O)₂-NH-(C₁-C₃)-Alkyl und S(O)₂-N((C₁-C₃)-Alkyl)₂, substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere Ring-Heteroatome aus der Reihe N, O und S enthalten;
Heterocyclyl für den Rest eines monocyclischen oder polycyclischen, 5-gliedrigen bis 11-gliedrigen gesättigten oder teilweise ungesättigten Heterocyclus steht, der ein oder mehrere Ring-Heteroatome aus der Reihe N, O und S enthält und der durch einen oder mehrere gleiche oder verschiedene Substituenten aus der Reihe Fluor, (C₁-C₅)-Alkyl, OH, -O-(C₁-C₅)-Alkyl, -O-(C₂-C₄)-Alkyl-O-(C₁-C₃)-Alkyl, NH₂, -NH-(C₁-C₃)-Alkyl, -N((C₁-C₃)-Alkyl)₂, CN, CO-NH₂, CO-NH-(C₁-C₃)-Alkyl, CO-N((C₁-C₃)-Alkyl)₂, COOH und CO-O-(C₁-C₅)-Alkyl substituiert sein kann;
n für 0, 1 oder 2 steht;
m für 2, 3 oder 4 steht;
in allen ihren stereoisomeren Formen und Mischungen davon in allen Verhältnissen, und/oder ihrer physiologisch verträglichen Salze zur Herstellung eines Medikaments zur Aktivierung der löslichen Guanylatcyclase.

Können Gruppen oder Substituenten mehrfach in den Verbindungen der Formel I vorkommen, so können sie alle unabhängig voneinander die angegebenen Bedeutungen haben und können jeweils gleich oder verschieden sein.

Alkylreste können geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind. Beispiele für Alkylgruppen sind Methyl, Ethyl, Propyl, Butyl, Pentyl, Hexyl, Heptyl, Octyl, Nonyl, Decyl, die n-Isomeren dieser Reste, Isopropyl, Isobutyl, Isopentyl, sec-Butyl, tert-Butyl, Neopentyl, 3,3-Dimethylbutyl.

Phenylreste, Naphthylreste und heterocyclische Reste, zum Beispiel Heteroarylreste, können, soweit nicht anders angegeben, unsubstituiert sein oder einen oder mehrere, zum Beispiel ein, zwei, drei oder vier, gleiche oder verschiedene Substituenten tragen, die sich in beliebigen Positionen befinden können. Soweit nicht anders angegeben, können in diesen Resten als Substituenten zum Beispiel die in der Definition der Gruppe Aryl angegebenen Substituenten auftreten. Sind in Verbindungen der Formel I Nitrogruppen als Substituenten vorhanden, so können insgesamt nur bis zu zwei Nitrogruppen im Molekül vorhanden sein. Sind zum Beispiel in Arylresten wie zum Beispiel Phenylresten und/oder in heterocyclischen Resten als Substituenten Phenylreste, Phenoxyreste, Benzylreste oder Benzyloxyreste vorhanden, so kann in diesen der Benzolring, auch wiederum unsubstituiert sein oder durch einen oder mehrere, zum Beispiel ein, zwei, drei oder vier, gleiche oder verschiedene Reste substituiert sein, zum Beispiel durch Reste aus der Reihe (C₁-C₄)-Alkyl, Halogen, Hydroxy, (C₁-C₄)-Alkoxy, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino.

In monosubstituierten Phenylresten kann sich der Substituent in der 2-Position, der 3-Position oder der 4-Position befinden, in disubstituierten Phenylresten können sich die Substituenten in 2,3-Position, 2,4-Position, 2,5-Position, 2,6-Position, 3,4-Position oder 3,5-Position befinden. In trisubstituierten Phenylresten können sich die Substituenten in 2,3,4-Position, 2,3,5-Position, 2,3,6-Position, 2,4,5-Position, 2,4,6-Position oder 3,4,5-Position befinden. Tolyl (= Methylphenyl) ist 2-Tolyl, -3-Tolyl oder 4-Tolyl. Naphthyl kann 1-Naphthyl oder 2-Naphthyl sein. In monosubstituierten 1-Naphthylresten kann sich der Substituent in der 2-Position, der 3-Position, der 4-Position, der 5-Position, der 6-Position, der 7-Position oder der 8-Position befinden, in monosubstituierten 2-Naphthylresten in der 1-Position, der 3-Position, der 4-Position, der 5-Position, der 6-Position, der 7-Position oder der 8-Position. Auch in höher substituierten Naphthylresten, zum Beispiel 1-Naphthylresten und 2-Naphthylresten, die zwei oder drei Substituenten tragen, können sich die Substituenten in allen möglichen Positionen befinden.

Heteroarylreste, Heterocyclylreste und Ringe, die von zwei an ein Stickstoffatom gebundenen Gruppen zusammen mit diesem Stickstoffatom gebildet werden, leiten sich bevorzugt von Heterocyclen ab, die ein, zwei, drei oder vier gleiche oder verschiedene Ring-Heteroatome enthalten, besonders bevorzugt von Heterocyclen, die ein oder zwei oder drei, insbesondere ein oder zwei, gleiche oder verschiedene Heteroatome enthalten. Soweit nicht anders angegeben, können die Heterocyclen monocyclisch oder polycyclisch sein, zum Beispiel monocyclisch, bicyclisch oder tricyclisch. Bevorzugt sind sie monocyclisch oder bicyclisch. Die Ringe sind bevorzugt 5-Ringe, 6-Ringe oder 7-Ringe. Beispiele für monocyclische und bicyclische heterocyclische Systeme, von denen sich in den Verbindungen der Formel I vorkommende Reste ableiten können, sind Pyrrol, Furan, Thiophen, Imidazol, Pyrazol, 1,2,3-Triazol, 1,2,4-Triazol, 1,3-Dioxol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin, 1,3-Thiazepin, Indol, Benzothiophen, Benzofuran, Benzothiazol, Benzimidazol, Chinolin, Isochinolin, Cinnolin, Chinazolin, Chinoxalin, Phthalazin, Thienothiophene, 1,8-Naphthyridin und andere Naphthyridine, Pteridin, oder Phenothiazin, alle jeweils in gesättigter Form (Perhydro-Form) oder in teilweise ungesättigter Form (zum Beispiel Dihydro-Form und Tetrahydro-Form) oder in maximal ungesättigter Form, soweit die betreffenden Formen bekannt und stabil sind. Zu den in Betracht kommenden Heterocyclen gehören somit beispielsweise auch die gesättigten Heterocyclen Pyrrolidin, Piperidin, Piperazin, Morpholin und Thiomorpholin. Der Sättigungsgrad von heterocyclischen Gruppen ist bei den einzelnen Definitionen angegeben. Ungesättigte Heterocyclen können zum Beispiel eine, zwei oder drei Doppelbindungen im Ringsystem enthalten. 5-Ringe und 6-Ringe in monocyclischen und polycyclischen Heterocyclen können insbesondere auch aromatisch sein.

Die von diesen Heterocyclen abgeleiteten Reste können über jedes geeignete Kohlenstoffatom gebunden sein. Stickstoffheterocyclen, die an einem RingStickstoffatom ein Wasserstoffatom (oder einen Substituenten) tragen, zum Pyrrol, Imidazol, Pyrrolidin, Morpholin, Piperazin etc., können auch über ein RingStickstoffatom gebunden sein, insbesondere, wenn der betreffende Stickstoffheterocyclus an ein Kohlenstoffatom gebunden ist. Ein Thienylrest kann beispielsweise als 2-Thienylrest oder 3-Thienylrest vorliegen, ein Furanrest als 2-Furylrest oder 3-Furylrest, ein Pyridylrest als 2-Pyridylrest, 3-Pyridylrest oder 4-Pyridylrest, ein Piperidinrest als 1-Psperidyirest, 2-Piperidylrest, 3-Piperidylrest oder 4-Piperidylrest, ein Thiomorpholinrest als 2-Thiomorpholinylrest, 3-Thiomorpholinylrest oder 4-Thiomorpholinylrest (= Thiomorpholinorest). Ein über ein Kohlenstoffatom gebundener Rest, der sich vom 1,3-Thiazol oder vom Imidazol ableitet, kann über die 2-Position, die 4-Position oder die 5-Position gebunden sein.

Soweit nicht anders angegeben, können die heterocyclischen Gruppen, wie zum Beispiel Heteroarylreste, unsubstituiert sein oder einen oder mehrere, zum Beispiel einen, zwei, drei oder vier, gleiche oder verschiedene Substituenten tragen. Die Substituenten in Heterocyclen können sich in beliebigen Positionen befinden, beispielsweise in einem 2-Thienylrest oder 2-Furylrest in der 3-Position und/oder in der 4-Position und/oder in der 5-Position, in einem 3-Thienylrest oder 3-Furylrest in der 2-Position und/oder in der 4-Position und/oder in der 5-Position, in einem 2-Pyridylrest in der 3-Position und/oder in der 4-Position und/oder in der 5-Position und/oder in der 6-Position, in einem 3-Pyridylrest in der 2-Position und/oder in der 4-Position und/oder in der 5-Position und/oder in der 6-Position, in einem 4-Pyridylrest in der 2-Position und/oder in der 3-Position und/oder in der 5-Position und/oder in der 6-Position. Soweit nicht anders angegeben, können als Substituenten zum Beispiel die in der Definition der Gruppe Aryl angegebenen Substituenten auftreten, im Falle von gesättigten oder teilweise ungesättigten Heterocyclen als weiterere Substituenten auch die Oxogruppe und die Thioxogruppe. Substituenten an einem Heterocyclus wie auch Substituenten an einem Carbocyclus können auch einen Ring bilden, es können also an ein Ringsystem weitere Ringe ankondensiert sein, so daß zum Beispiel cyclopenta-kondensierte, cyclohexa-kondensierte oder benzokondensierte Ringe vorliegen können. Als Substituenten an einem substituierbaren Stickstoffatom eines Heterocyclus kommen insbesondere zum Beispiel unsubstituierte (C₁-C₅)-Alkylreste und arylsubstituierte Alkylreste, Arylreste, Acylreste wie CO-(C₁-C₅)-Alkyl, oder Sulfonylreste wie SO₂-(C₁-C₅)-Alkyl in Betracht. Geeignete Stickstoffheterocyclen können auch als N-Oxide oder als Quartärsalze mit einem von einer physiologisch verträglichen Säure abgeleiteten Anion als Gegenion vorliegen. Pyridylreste können zum Beispiel als Pyridin-N-oxide vorliegen.

Halogen bedeutet Fluor, Chlor, Brom oder Iod, vorzugsweise Fluor oder Chlor.

Die Formel la, in der A¹, R² und R³ die oben für die Formel I angegebenen Bedeutungen haben, gibt die oben definierten Verbindungen der Formel I, in der A² für einen Benzolring steht, wieder.

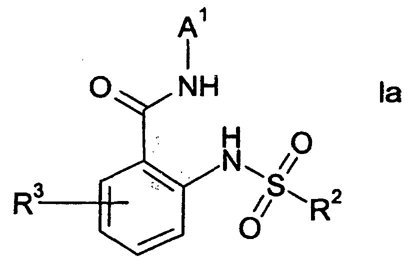

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Die Erfindung umfaßt alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere werden also in enantiomerenreiner Form, sowohl als linksdrehende als auch als rechtsdrehende Antipoden, in Form von Racematen und in Form von Mischungen der beiden Enantiomeren in allen Verhältnissen von der Erfindung umfaßt. Bei Vorliegen einer cis/trans-Isomerie werden sowohl die cis-Form als auch die trans-Form und Gemische dieser Formen in allen Verhältnissen von der Erfindung umfaßt. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I.

Enthalten die Verbindungen der Formel I eine oder mehrere saure oder basische Gruppen, so umfaßt die Erfindung auch die entsprechenden physiologisch oder toxikologisch verträglichen Salze, insbesondere die pharmazeutisch verwendbaren Salze. So können die Verbindungen der Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, 8emsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine (Zwitterionen) zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die vorliegende Erfindung umfaßt weiterhin alle Solvate von Verbindungen der Formel I, zum Beispiel Hydrate oder Addukte mit Alkoholen.

A¹ steht bevorzugt für Phenyl, das durch einen oder mehrere gleiche oder verschiedene Reste R¹ substituiert sein kann.

A¹ kann unsubstituiert sein, das heißt nur Wasserstoffatome tragen, oder kann wie angegeben durch einen oder mehrere, zum Beispiel einen, zwei, drei oder vier, gleiche oder verschiedene Reste R¹ substituiert sein. Bevorzugt ist ein substituierter Rest A¹ durch einen, zwei oder drei, insbesondere einen oder zwei, Reste R¹ substituiert. Reste R¹ sind bevorzugt an Kohlenstoffatome in A¹ gebunden, die dem Kohlenstoffatom, das die Gruppe CO-NH trägt, nicht direkt nachbart sind. Steht A¹ für Phenyl, so stehen Reste R¹ besonders bevorzugt in meta-Position und/oder in para-Position, bezogen auf das Kohlenstoffatom, das die Gruppe CO-NH trägt. Trägt ein für A¹ stehender Phenylrest einen Rest R¹, so steht dieser in vielen Fällen besonders vorteilhaft in der para-Position. Trägt ein für A¹ stehender Phenylrest als Rest R¹ eine Trifluormethylgruppe, so steht diese bevorzugt in der meta-Position. Trägt ein für A¹ stehender Phenylrest zwei für Chlor stehende Reste R¹, so stehen die beiden Chloratome bevorzugt in den Positionen 3 und 4.

Bevorzugte Reste R¹ sind Halogen, insbesondere Chlor, Trifluormethyl, (C₃-C₇)-Alkyl, Carboxymethyl, CONR⁵R⁶, 5-gliedriges bis 7-gliedriges Heterocyclyl, -O-Aryl, -CO-(C₁-C₁₀)-Alkyl, -CO-Aryl, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-Aryl)₂ und -N(SO₂-(C₁-C₁₀)-Alkyl)₂, wobei alle Alkylreste durch einen oder mehrere gleiche oder verschiedene Reste R⁴ substituiert sein können. In einer für R¹ stehenden Gruppe CONR⁵R⁶ bilden bevorzugt R⁵ und R⁶ zusammen mit dem Stickstoffatom, an das sie gebunden sind, einen 5-gliedrigen bis 8-gliedrigen gesättigten oder teilweise ungesättigten Ring, der zusätzlich zu dem die Gruppen R⁵ und R⁶ tragenden Stickstoffatom noch ein oder mehrere weitere Ring-Heteroatome aus der Reihe N, O und S enthalten kann und der wie oben angegeben durch einen oder mehrere gleiche oder verschiedene Substituenten substituiert sein kann. Eine Gruppe besonders bevorzugter Reste R¹ bilden die Reste Halogen, insbesondere Chlor, Trifluormethyl, -O-Aryl, -NH-CO-(C₁-C₁₀)-Alkyl, -NH-CO-Aryl, -NH-SO₂-(C₁-C₁₀)-Alkyl, und -NH-SO₂-Aryl, wobei alle Alkylreste durch einen oder mehrere gleiche oder verschiedene Reste R⁴ substituiert sein können. Eine weitere Gruppe besonders bevorzugter Reste R¹ bilden die Reste CONR⁵R⁶, -CO-(C₁-C₁₀)-Alkyl, -CO-Aryl, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-Aryl)₂ und -N(SO₂-(C₁-C₁₀)-Alkyl)₂, wobei alle Alkylreste durch einen oder mehrere gleiche oder verschiedene Reste R⁴ substituiert sein können.

R² steht bevorzugt für Phenyl, das durch einen oder zwei gleiche oder verschiedene Reste aus der Reihe Halogen, CF₃ und (C₁-C₃)-Alkyl substituiert sein kann.

Der für A² stehende Benzolring kann unsubstituiert sein, das heißt nur für Wasserstoff stehendes R³ tragen, oder wie angegeben substituiert sein, das heißt einen oder mehrere von Wasserstoff verschiedene Reste R³ tragen. Die anderen Substituentenpositionen am Ring A², die keinen von Wasserstoff verschiedenen Rest R³ tragen, tragen Wasserstoffatome. Trägt der Ring A² einen oder mehrere Reste R³, die von Wasserstoff verschieden sind, so trägt er bevorzugt einen oder zwei derartige Reste R³, insbesondere einen derartigen Rest R³. Derartige Reste R³ stehen bevorzugt in solchen Positionen des Ringes A², die den Gruppen CO-NH und R²SO₂-NH nicht direkt nachbart sind. Bevorzugt steht derartiges R³ für (C₁-C₃)-Alkyl, Methoxy, Halogen oder CF₃, besonders bevorzugt für Methyl oder Chlor. Ganz besonders bevorzugt ist es, wenn der für A² stehende Benzolring als Substituenten ein Chloratom trägt, das heißt, wenn ein für Chlor stehender Rest R³ vorhanden ist und die anderen Substituentenpositionen am Benzolring Wasserstoffatometragen. In dem für A² stehenden Benzolring stehen von Wasserstoff verschiedene Reste R³ bevorzugt in den Positionen 4 und/oder 5. Ist nur ein derartiger Rest R³ an dem für A² stehenden Benzolring vorhanden, so steht dieser Rest bevorzugt in der Position 5 (bezogen auf die Gruppe CO-NH in der 1-Position).

Wenn eine Gruppe durch einen oder mehrere Reste R⁴ substituiert ist, so ist sie bevorzugt durch einen, zwei oder drei, insbesondere einen oder zwei, gleiche oder verschiedene Reste R⁴ substituiert. R⁴ steht bevorzugt für Hydroxy, (C₁-C₄)-Alkyloxy, Di-((C₁-C₄)-alkyl)amino oder Heteroaryl.

R⁵ und R⁶ stehen bevorzugt unabhängig voneinander für Wasserstoff, (C₁-C₉)-Alkyl, -(C₁-C₃)-Alkyl-O-(C₁-C₄)-Alkyl oder 5-gliedriges oder 6-gliedriges Aryl oder bilden zusammen mit dem R⁵ und R⁶ tragenden Stickstoffatom einen 5-gliedrigen bis 7-gliedrigen Heterocyclus, der zusätzlich zu dem die Gruppen R⁵ und R⁶ tragenden Stickstoffatom noch ein weiteres Ring-Heteroatom aus der Reihe N, O und S enthalten kann und der durch einen oder mehrere, zum Beispiel einen, zwei, drei oder vier, gleiche oder verschiedene Reste wie oben angegeben substituiert sein kann, insbesondere durch Reste aus der Reihe (C₁-C₃)-Alkyl, 5-gliedriges Aryl und 6-gliedriges Aryl. Besonders bevorzugt bilden R⁵ und R⁶ zusammen mit dem sie tragenden Stickstoffatom einen 5-gliedrigen bis 7-gliedrigen Heterocyclus, der zusätzlich zu dem die Gruppen R⁵ und R⁶ tragenden Stickstoffatom noch ein weiteres Ring-Heteroatom aus der Reihe N, O und S enthalten kann und der durch einen oder mehrere, zum Beispiel einen, zwei, drei oder vier gleiche oder verschiedene Reste wie oben angegeben substituiert sein kann, insbesondere durch Reste aus der Reihe (C₁-C₃)-Alkyl, 5-gliedriges Aryl und 6-gliedriges Aryl. Ganz besonders bevorzugt leitet sich ein solcher Ring, der von R⁵ und R⁶ zusammen mit dem sie tragenden Stickstoffatom gebildet wird, ab vom Morpholin, Thiomorpholin, 1,1-Dioxo-thiomorpholin, 1-Oxo-thiomorpholin, 3,5-Dimethylmorpholin, cis-3,5-Dimethylmorpholin, 1-(Pyrimidin-2-yl)-piperazin, Piperidin-4-carbonsäureamid, 1-(2-Hydroxyethyl)-piperazin, 1-Methylpiperazin, 1-Ethylpiperazin, von 1-Arylpiperazinen, vom Piperazin-1-carbonsäureethylester, Piperidin, 2-Methylpiperidin, 4-Hydroxypiperidin, vom 4-Oxopiperidin oder einem Ketal davon wie 1,4-Dioxa-8-azaspiro[4.5]decan, vom Tetrahydropyridin, Tetrahydropyrimidin, 1-Methylhomopiperazin, Thiazolidin, Pyrrolin, 3-Hydroxypyrrolidin, 1,2,3,4-Tetrahydroisochinolin oder 2,3-Dihydro-1H-isoindol, wobei der Ring über das RingStickstoffatom bzw. im Falle der Piperazinderivate über das nicht substituierte RingStickstoffatom gebunden ist. Speziell bevorzugt leitet sich ein solcher Ring, der von R⁵ und R⁶ zusammen mit dem sie tragenden Stickstoffatom gebildet wird, ab vom Morpholin, Thiomorpholin, 1,1-Dioxo-thiomorpholin, 1-Oxo-thiomorpholin, 3,5-Dimethylmorpholin, cis-3,5-Dimethylmorpholin, 1-(Pyrimidin-2-yl)-piperazin, Piperidin-4-carbonsäureamid, 1,2,3,4-Tetrahydroisochinolin oder 2,3-Dihydro-1Hisoindol.

R⁸ steht bevorzugt für Wasserstoff, (C₁-C₃)-Alkyl, Di-((C₁-C₄)-alkyl)amino oder -(C₁-C₃)-Alkyl-O-(C₁-C₄)-Alkyl.

R⁹ steht bevorzugt für Wasserstoff, (C₁-C₃)-Alkyl oder Acetyl.

Aryl bedeutet bevorzugt Phenyl oder Heteroaryl, insbesondere Phenyl oder 5-gliedriges oder 6-gliedriges Heteroaryl. Bevorzugte Substituenten in Arylresten sind Halogen, CF₃, (C₁-C₃)-Alkyl, Cyan, Nitro und (C₁-C₃)-Alkyloxy, besonders bevorzugte Substituenten sind CF₃, Chlor, Methyl und Methoxy.

Heteroaryl steht bevorzugt für Reste, die sich von den Heteroaromaten Thiophen, Pyrazol, Thiazol, Oxazol, Pyridin, Pyrimidin, Pyridazin und Tetrazol ableiten.

Heterocyclyl steht bevorzugt für Reste, die sich von gesättigten Heterocyclen ableiten, insbesondere für Reste, die sich vom Pyrrolidin, Piperidin, von N-Alkylpiperazinen, vom Morpholin, von Dialkylmorpholinen, vom Thiomorpholin oder Tetrahydrofuran ableiten.

Wenn eine Gruppe S(O)ₙ an ein Stickstoffatom gebunden ist, steht darin die Zahl n bevorzugt für 1 oder 2, besonders bevorzugt für 2.

Bevorzugte Verbindungen gemäß der vorliegenden Erfindung sind Verbindungen der Formel I, in der einer oder mehrere der darin enthaltenen Reste bevorzugte Bedeutungen haben, wobei alle Kombinationen von bevorzugten Substituentendefinitionen Gegenstand der vorliegenden Erfindung sind. Auch von allen bevorzugten Verbindungen der Formel I betrifft die vorliegende Erfindung alle ihre stereoisomeren Formen und Mischungen davon in allen Verhältnissen, und ihre physiologisch verträglichen Salze. Eine Gruppe von bevorzugten Verbindungen gemäß der vorliegenden Erfindung bilden zum Beispiel solche Verbindungen der Formel I, in der A¹ für Phenyl steht, das in der 4-Position einen Rest R¹ trägt; der Ring A², der die Kohlenstoffatome umfaßt, die die Gruppen CO-NH- und NH-SO₂R² tragen, für einen Benzolring steht; und R¹ für einen Substituenten aus der Reihe CO-(C₁-C₁₀)-Alkyl, CO-Aryl, CO-NR⁵R⁶, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-(C₁-C₁₀)-Alkyl)₂, und -N(SO₂-Aryl)₂, worin alle Alkylreste durch einen oder mehrere gleiche oder verschiedene Reste R⁴ substituiert sein können, steht; in allen ihren stereoisomeren Formen und Mischungen davon in allen Verhältnissen, und ihre physiologisch verträglichen Salze.

Verbindungen der Formel I können nach oder analog zu in der Literatur beschriebenen Verfahren hergestellt werden. Auf den entsprechenden Inhalt von Dokumenten, in denen bereits Verbindungen der Formel I beschrieben sind, zum Beispiel die DE-A-35 23 705 und ihre Äquivalenzen, wird hier ausdrücklich Bezug genommen. Der entsprechende Inhalt dieser Dokumente ist Bestandteil der vorliegenden Offenbarung. Die Herstellung von Verbindungen der Formel I ist zudem im folgenden erläutert.

Gemäß dem Schema 1 kann zunächst eine Aminocarbonsäure der Formel II in einem Lösungsmittel wie Wasser, Pyridin oder einem Ether in Gegenwart einer Base mit einem Sulfonylchlorid der Formel R²-SO₂-Cl oder einem Sulfonsäureanhydrid umgesetzt werden. Als Base kommen anorganische Basen wie zum Beispiel

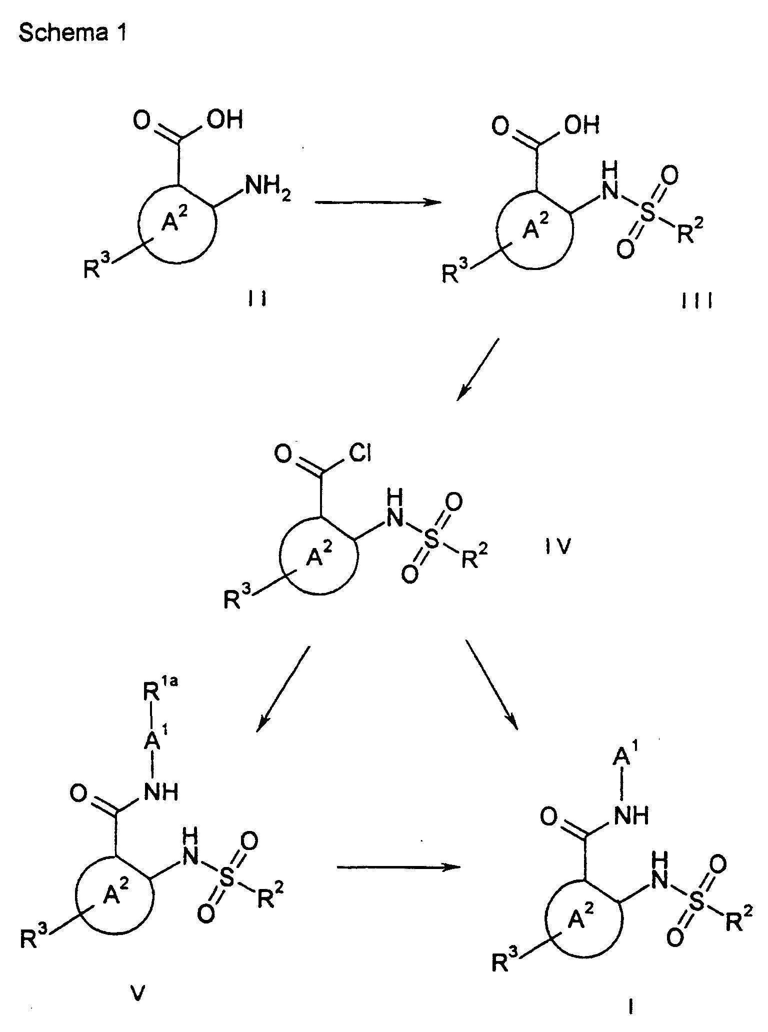

Natriumcarbonat oder organische Basen wie zum Beispiel Pyridin oder Triethylamin in Betracht. Die erhaltene Sulfonylaminocarbonsäure der Formel III kann dann zum Beispiel durch Umsetzung mit einem Chlorierungsagenz wie zum Beispiel Phosphorpentachlorid, Phosphoroxychlorid oder Thionylchlorid in einem inerten Lösungsmittel zu einem Säurechlorid der Formel IV aktiviert werden und dann mit einem Arylamin umgesetzt werden. Die Aktivierung der Carbonsäuregruppe in der Verbindung der Formel III kann aber auch auf andere Art erfolgen, zum Beispiel durch eine der zahlreichen, dem Fachmann geläufigen Methoden, die in der Peptidchemie zur Knüpfung von Amidbindungen angewandt werden, zum Beipiel durch Überführung in ein gemischtes Anhydrid oder einen aktivierten Ester oder unter Verwendung eines Carbodiimids wie Dicyclohexylcarbodiimid.
Die Umsetzung der aktivierten Sulfonylaminocarbonsäure mit einem Arylamin wird vorteilhaft in einem inerten Lösungsmittel wie zum Beispiel Pyridin, Tetrahydrofuran oder Toluol mit ohne ohne Zusatz einer inerten Hilfsbase, zum Beispiel eines tertiären Amins oder von Pyridin, durchgeführt. Enthält das in die Umsetzung mit der aktivierten Carbonsäure eingesetzte Arylamin in der Gruppe A¹ bereits den oder die gewünschten Substituenten R¹, hat das Arylamin also die Formel A¹-NH₂, worin die Gruppe A¹ wie oben angegeben einen oder mehrere Substituenten R¹ enthalten kann, so führt die Umsetzung direkt zum Endprodukt der Formel I. Die aktivierten Carbonsäuren können aber auch zunächst mit einem Arylamin der Formel R^{1a}-A¹-NH₂ umgesetzt werden, in der R^{1a} für Wasserstoff oder für eine oder mehrere der Gruppen R¹ steht, die als Substituenten in A¹ enthalten sein können, oder R^{1a} für eine oder mehrere Gruppen steht, die in Gruppen R¹ gemäß der obigen Definition umgewandelt werden können. Beispielsweise kann R^{1a} für ein Wasserstoffatom stehen, das in einer elektrophilen Reaktion durch einen anderen Rest wie zum Beispiel ein Halogenatom oder eine Aldehydgruppe ersetzt wird, oder für eine Estergruppe, die in eine Amidgruppe überführt wird. Die Überführung des zunächst erhaltenen Reaktionsprodukts der Formel V in eine Verbindung der Formel I kann nach Standardverfahren erfolgen.

Verbindungen der Formel I können zum Beispiel auch dadurch erhalten werden, daß zunächst eine geeignet substituierte Nitrocarbonsäure der Formel VI aktiviert wird, zum Beispiel durch Überführung in das entsprechende Säurechlorid der Formel VII oder auf andere Art, und dann zum Beispiel mit einem substituierten Arylamin der Formel A¹-NH₂ analog den oben beschriebenen Verfahren umgesetzt wird (siehe Schema 2). Bevor in den erhaltenen Nitro-Zwischenprodukten der Formel VIII die

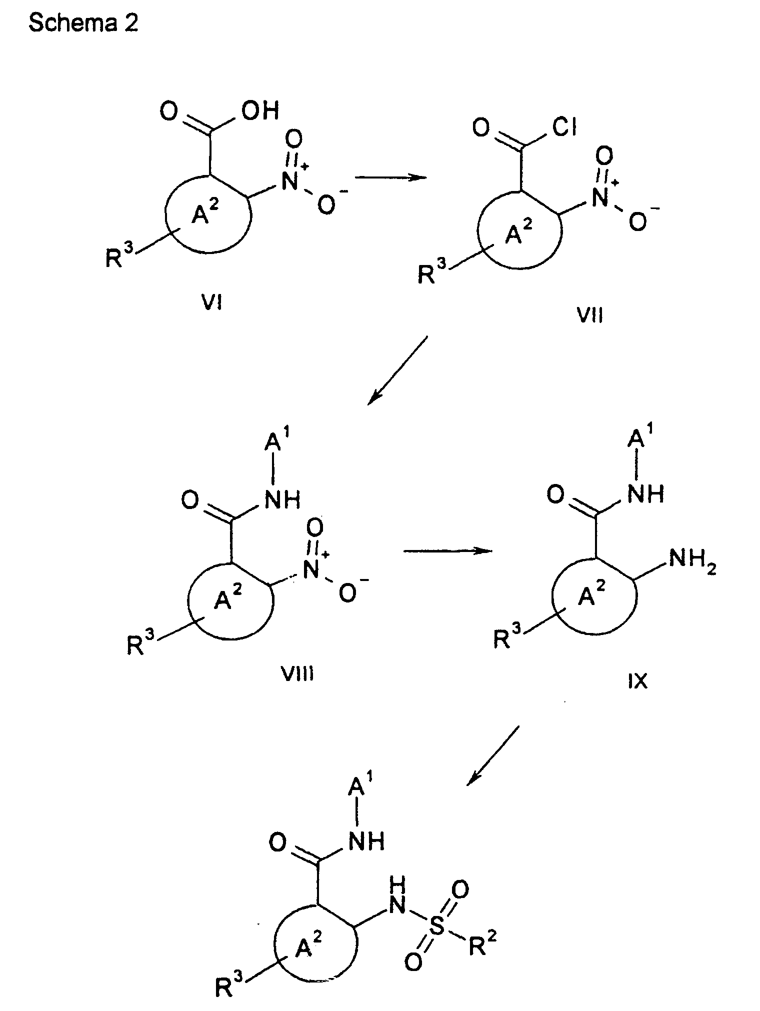

Nitrogruppe zur Aminogruppe reduziert wird, kann die aktivierende Wirkung der Nitrogruppe auf den Ring A² ausgenutzt werden und ein geeigneter Rest R³, zum Beispiel ein Halogenatom, durch Umsetzung mit einem Nucleophil, zum Beispiel einem Amin, durch einen anderen Rest R³ ausgetauscht werden. Die Reduktion der Nitrogruppe zur Aminogruppe kann beispielsweise durch katalytische Hydrierung in Gegenwart eines Edelmetall-Katalysators oder vorzugsweise in Gegenwart von Raney-Nickel in einem Lösungsmittel wie Ethanol, Eisessig oder ethanolischer Salzsäure erfolgen, oder durch Reduktion mit einem unedlen Metall wie Zinn, Zink oder Eisen in Gegenwart von Säure. Die Reduktion kann beispielsweise auch mit Zinn-(II)-chlorid oder durch Reaktion mit Natriumdithionit, vorteilhaft zum Beispiel in einem Gemisch von Methanol, Tetrahydrofuran und Wasser als Lösungsmittel, vorgenommen werden. Die Sulfonylierung der Aminogruppe in dem Reduktionsprodukt der Formel IX mit einem aktivierten Sulfonsäurederivat analog den oben beschriebenen Reaktionen, zum Beispiel mit einem Sulfonsäurechlorid in Gegenwart von Pyridin, ergibt schließlich die Verbindung der Formel I. An Stelle eines Arylamins der Formel A¹-NH₂ kann auch wiederum ein Arylamin der Formel R^{1a}-A¹-NH₂ eingesetzt werden, in der R^{1a} die oben angegebene Bedeutung hat, und die Gruppe oder die Gruppen R^{1a} dann anschließend in die Gruppe oder die Gruppen R¹ überführt werden.

Alle Reaktionen zur Synthese der Verbindungen der Formel I sind dem Fachmann an sich wohlbekannt und können unter Standardbedingungen durchgeführt werden. Nähere Angaben dazu finden sich zum Beispiel in Houben-Weyl, Methoden der Organischen Chemie, Thieme-Verlag, Stuttgart, oder Organic Reactions, John Wiley & Sons, New York. Je nach den Gegebenheiten des Einzelfalls kann es bei der Synthese der Verbindungen der Formel I zur Vermeidung von Nebenreaktionen auch vorteilhaft oder notwendig sein, bestimmte funktionelle Gruppen vorübergehend durch die Einführung von Schutzgruppen zu blockieren und später dann wieder freizusetzen oder funktionelle Gruppen zunächst in Form von Vorstufen einzusetzen, aus denen in einem späteren Schritt dann die gewünschte funktionelle Gruppe erzeugt wird. Solche Synthesestrategien und die für den Einzelfall geeigneten Schutzgruppen oder Vorstufen sind dem Fachmann bekannt. Die erhaltenen Verbindungen der Formel I können gegebenenfalls nach üblichen Reinigungsmethoden, zum Beispiel durch Umkristallisation oder Chromatographie, gereinigt werden. Die Ausgangsverbindungen für die Herstellung der Verbindungen der Formel I sind käuflich erhältlich oder können nach oder analog zu Literaturvorschriften hergestellt werden.

Die Verbindungen der Formel I bewirken über die Aktivierung der löslichen Guanylatcyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung oder Normalisierung des vorhandenen cGMP-Spiegels angestrebt wird. Die Aktivierung der sGC durch die Verbindungen der Formel I kann zum Beispiel in dem unten beschriebenen Aktivitätsassay untersucht werden.

Krankheiten und pathologische Zustände, die mit einem niedrigen cGMP-Spiegel verbunden sind oder bei denen eine Erhöhung oder Normalisierung des cGMP-Spiegels angestrebt wird und zu deren Therapie und Prophylaxe Verbindungen der Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie zur Verbesserung einer eingeschränkten Lemfähigkeit oder Gedächtnisleistung. Bevorzugt werden die Verbindungen der Formel I bei Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, bei erektiler Dysfunktion oder zur Verbesserung einer eingeschränkten Lernfähigkeit oder Gedächtnisleistung eingesetzt.

Die Verbindungen der Formel I und ihre physiologisch verträglichen Salze können somit am Tier, bevorzugt am Säugetier, und insbesondere am Menschen als Arzneimittel für sich allein, in Mischungen untereinander oder zusammen mit anderen Wirkstoffen verwendet werden. Gegenstand der vorliegenden Erfindung ist daher insbesondere auch die Verwendung von Verbindungen der Formel I und ihren physiologisch verträglichen Salzen zur Herstellung eines Medikaments zur Therapie oder Prophylaxe der vorstehend genannten Krankheitsbilder sowie die Verwendung zur Herstellung eines Medikaments zur Erhöhung oder Normalisierung eines gestörten cGMP-Haushalts. Ebenso ist Gegenstand der Erfindung die Verwendung der Verbindungen der Formel I und ihrer physiologisch verträglichen Salze zur Aktivierung der löslichen Guanylatcyclase, ihre Verwendung zur Therapie oder Prophylaxe der vorstehend genannten Krankheitsbilder und ihre Verwendung zur Erhöhung oder Normalisierung eines gestörten cGMP-Haushalts.

Arzneimittel gemäß der vorliegenden Erfindung können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die Herstellung der erfindungsgemäßen Medikamente kann nach den bekannten Standardverfahren zur Herstellung pharmazeutischer Präparate erfolgen. Dazu werden ein oder mehrere Verbindungen der Formel I und/oder ihre physiologisch verträglichen Salze zusammen mit einem oder mehreren festen oder flüssigen galenischen Trägerstoffen und/oder Zusatzstoffen oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann. Die pharmazeutischen Präparate enthalten eine therapeutisch oder prophylaktisch wirksame Dosis der Verbindungen der Formel I und/oder ihrer physiologisch verträglichen Salze, die normalerweise 0.5 bis 90 Gewichtsprozent des pharmazeutischen Präparats ausmacht.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektionspräparaten oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung der angestrebten Wirkung angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 200 mg Wirkstoff der Formel I und/oder dessen physiologisch verträgliche Salze.

Die Verbindungen der Formel I aktivieren die lösliche Guanylatcyclase, vornehmlich durch Bindung in der Häm-Bindungstasche des Enzyms. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zellproben oder Gewebsproben. Femer können die Verbindungen der Formel I und ihre Salze, wie bereits oben angesprochen, als Zwischenprodukte zur Herstellung weiterer Arzneimittelwirkstoffe dienen.

Neben bereits bekannten Verbindungen umfaßt die Formel I mit der obigen allgemeinen Definition der Reste auch Verbindungen, die noch nicht beschrieben sind. Gegenstand der vorliegenden Erfindung sind auch die Verbindungen der Formel Ik, 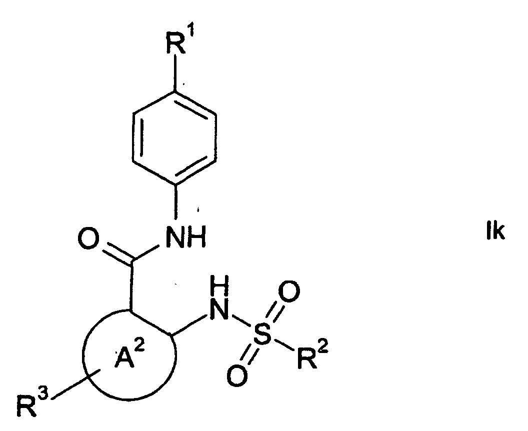 in der
der Ring A², der die Kohlenstoffatome umfaßt, die die Gruppen CO-NH- und NH-SO₂R² tragen, für einen Benzolring steht;
R¹ für einen Substituenten aus der Reihe CO-(C₁-C₁₀)-Alkyl, CO-Aryl, CO-NR⁵R⁶, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-(C₁-C₁₀)-Alkyl)₂, und -N(SO₂-Aryl)₂, worin alle Alkylreste durch einen oder mehrere gleiche oder verschiedene Reste R⁴ substituiert sein können, steht;
R² für Phenyl, das durch einen oder mehrere gleiche oder verschiedene Substituenten aus der Reihe Halogen, CF₃, (C₁-C₃)-Alkyl, Cyan, Nitro und (C₁-C₃)-Alkoxy substituiert sein kann, steht;
R³ für einen oder mehrere gleiche oder verschiedene Substituenten aus der Reihe Wasserstoff und Halogen steht;
R⁴ für Fluor, OH, -O-(C₁-C₁₀)-Alkyl, -O-(C₁-C₇)-Alkyl-R⁷, -O-Aryl, SH, -S-(C₁-C₁₀)-Alkyl, -S-(C₁-C₇)-Alkyl-R⁷, -S-Aryl, -P(O)(O-(C₁-C₅)-Alkyl)₂, -P(O)(OH)₂, CN, NR⁸R⁹, CO-NH₂, CO-NH-(C₁-C₃)-Alkyl, CO-N((C₁-C₃)-Alkyl)₂, COOH, CO-O-(C₁-C₅)-Alkyl, Heterocyclyl oder Oxo steht;
R⁵ für Wasserstoff, (C₁-C₁₀)-Alkyl, das durch einen oder mehrere gleiche oder verschiedene Substituenten R⁴ und/oder durch Aryl substituiert sein kann, Aryl, Heterocyclyl, CO-(C₁-C₁₀)-Alkyl, CO-Aryl, CO-Heterocyclyl, SO₂-(C₁-C₁₀)-Alkyl, SO₂-Aryl oder SO₂-Heterocyclyl steht;
R⁶ unabhängig von R⁵ eine der für R⁵ angegebenen Bedeutungen hat, oder R⁵ und R⁶ zusammen mit dem Stickstoffatom, an das sie gebunden sind, einen 5-gliedrigen bis 8-gliedrigen gesättigten oder teilweise ungesättigten Ring bilden, der zusätzlich zu dem die Gruppen R⁵ und R⁶ tragenden Stickstoffatom noch ein oder mehrere weitere Ring-Heteroatome aus der Reihe N, O und S enthalten kann und der durch einen oder mehrere gleiche oder verschiedene Substituenten aus der Reihe Fluor, (C₁-C₅)-Alkyl, (C₁-C₃)-Hydroxyalkyl, -(C₁-C₃)-Alkyl-O-(C₁-C₄)-Alkyl, Aryl, CF₃, OH, -O-(C₁-C₇)-Alkyl, -O-Aryl,-O-(C₂-C₄)-Alkyl-O-(C₁-C₇)-Alkyl, (C₂-C₃)-Alkylendioxy, NR⁸R⁹, CN, CO-NH₂, CO-NH-(C₁-C₃)-Alkyl, CO-N((C₁-C₃)-Alkyl)₂, COOH, CO-O-(C₁-C₅)-Alkyl, CHO, CO-(C₁-C₅)-Alkyl, S(O)ₙ-(C₁-C₄)-Alkyl, S(O)ₙ-NH₂, S(O)ₙ-NH-(C₁-C₃)-Alkyl, S(O)ₙ-N((C₁-C₃)-Alkyl)₂, Oxo, -(CH₂)ₘ-NH₂, -(CH₂)ₘ-NH-(C₁-C₄)-Alkyl und -(CH₂)ₘ-N((C₁-C₄)-Alkyl)₂ substituiert sein kann, wobei in dem Substituenten -(CH₂)ₘ-N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁷ für OH, -O-(C₁-C₇)-Alkyl, NH₂, -NH-(C₁-C₄)-Alkyl oder -N((C₁-C₄)-Alkyl)₂ steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁸ für Wasserstoff oder (C₁-C₇)-Alkyl, das durch einen oder mehrere gleiche oder verschiedene Substituenten aus der Reihe OH, -O-(C₁-C₅)-Alkyl, NH₂, -NH-(C₁-C₄)-Alkyl und -N((C₁-C₄)-Alkyl)₂ substituiert sein kann, steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁹ unabhängig von R⁸ eine der Bedeutungen von R⁸ hat oder für CO-(C₁-C₄)-Alkyl steht;
Aryl für Phenyl, Naphthyl oder Heteroaryl steht, die alle durch einen oder mehrere gleiche oder verschiedene Substituenten aus der Reihe Halogen, (C₁-C₅)-Alkyl, Phenyl, Tolyl, CF₃, NO₂, OH, -O-(C₁-C₅)-Alkyl, -O-(C₂-C₄)-Alkyl-O-(C₁-C₃)-Alkyl, (C₁-C₂)-Alkylendioxy, NH₂, -NH-(C₁-C₃)-Alkyl, -N((C₁-C₃)-Alkyl)₂, NH-CHO, -NH-CO-(C₁-C₅)-Alkyl, CN, CO-NH₂, CO-NH-(C₁-C₃)-Alkyl, CO-N((C₁-C₃)-Alkyl)₂, COOH, CO-O-(C₁-C₅)-Alkyl, Heterocyclyl, CHO, CO-(C₁-C₅)-Alkyl, S(O)ₙ-(C₁-C₄)-Alkyl, S(O)ₙ-Phenyl, S(O)ₙ-Tolyl, S(O)₂-NH₂, S(O)₂-NH-(C₁-C₃)-Alkyl und S(O)₂-N((C₁-C₃)-Alkyl)₂, substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere Ring-Heteroatome aus der Reihe N, O und S enthalten;
Heterocyclyl für den Rest eines monocyclischen oder polycyclischen, 5-gliedrigen bis 1 1-gliedrigen gesättigten oder teilweise ungesättigten Heterocyclus steht, der ein oder mehrere Ring-Heteroatome aus der Reihe N, O und S enthält und der durch einen oder mehrere gleiche oder verschiedene Substituenten aus der Reihe Fluor, (C₁-C₅)-Alkyl, OH, -O-(C₁-C₅)-Alkyl, -O-(C₂-C₄)-Alkyl-O-(C₁-C₃)-Alkyl, NH₂, -NH-(C₁-C₃)-Alkyl, -N((C₁-C₃)-Alkyl)₂, CN, CO-NH₂, CO-NH-(C₁-C₃)-Alkyl, CO-N((C₁-C₃)-Alkyl)₂, COOH und CO-O-(C₁-C₅)-Alkyl substituiert sein kann;
n für 0, 1 oder 2 steht;
m für 2, 3 oder 4 steht;
in allen ihren stereoisomeren Formen und Mischungen davon in allen Verhältnissen, und ihre physiologisch verträglichen Salze.

Alle Erläuterungen, die zu den Verbindungen der Formel I mit der zugehörigen, eingangs gegebenen Definition der Substituenten gemacht wurden, gelten, soweit anwendbar, entsprechend auch für die Verbindungen der Formel Ik mit der vorstehenden Definition der Substituenten. Dies trifft beispielsweise zu für die Tatsache, daß Gruppen und Substituenten, die mehrfach vorkommen, alte unabhängig voneinander sind, oder für alle Erläuterungen zu Alkylgruppen, Arylgruppen, heterocyclischen Resten, möglichen Substituenten, Salzen, Isomeren, Tautomeren, etc. Die vorliegende Erfindung umfaßt auch alle Salze der Verbindungen der Formel Ik, die sich wegen geringer physiologischer Verträglichkeit nicht direkt für eine Verwendung in Arzneimitteln, eignen, aber zum Beispiel als Zwischenprodukte für chemische Synthesen oder für die Herstellung physiologisch verträglicher Salze in Betracht kommen. Wie die Verbindungen der Formel I können auch die Verbindungen der Formel Ik durch die Formel la wiedergegeben werden. Auch alle bevorzugten Bedeutungen für die Reste in der Formel I gelten entsprechend für die Reste in der Formel Ik. Ebenso sind bevorzugte Verbindungen der Formel Ik solche Verbindungen, in denen einer oder mehrere der Reste bevorzugte Bedeutungen haben, wobei alle Kombinationen von bevorzugten Substituentendefinitionen Gegenstand der vorliegenden Erfindung sind.

Weiterhin gelten die obigen Angaben zur Herstellung der Verbindungen der Formel I und zu ihren biologischen Eigenschaften und zu ihrer Verwendung sowie zu sie enthaltenden pharmazeutischen Präparaten naturgemäß auch für die Verbindungen der Formel Ik. Gegenstand der vorliegenden Erfindung sind auch Verfahren zur Herstellung der vorstehend definierten Verbindungen der Formel Ik nach den oben beschriebenen Syntheseverfahren, die Verbindungen der Formel Ik und ihre physiologisch verträglichen Salze zur Anwendung als Arzneimittel und pharmazeutische Präparate, die eine wirksame Dosis mindestens einer Verbindung der Formel Ik oder eines physiologisch verträglichen Salzes davon als aktiven Bestandteil neben üblichen pharmazeutisch einwandfreien Trägerstoffen und/oder Zusatzstoffen enthalten.

Die nachfolgenden Beispielverbindungen, die nach oder analog zu in der Literatur beschriebenen Verfahren hergestellt wurden, erläutern die Erfindung, ohne sie einzuschränken.

### Beispiele

1) 2-(4-Chlor-phenylsulfonylamino)-N-(3-trifluormethyl-phenyl)-benzamid
   Schmp.: 169 °C
2) 5-Brom-2-(4-chlor-phenylsulfonylamino)-N-(3-trifluormethyl-phenyl)-benzamid
   Schmp.: 220 °C
3) 2-(4-Chlor-phenylsulfonylamino)-N-(2-naphthyl)-benzamid
   Schmp.: 189 °C
4) 5-Chlor-2-(4-chlor-phenylsulfonylamino)-N-(3-trifluormethyl-phenyl)-benzamid
   Schmp.: 216 °C
5) 5-Chlor-2-(4-chlor-phenylsulfonylamino)-N-(4-phenoxy-phenyl)-benzamid
   Schmp.: 205 °C
6) 5-Chlor-2-(4-chlor-phenylsulfonylamino)-N-(4-(4-chlor-phenoxy)-phenyl)-benzamid
   Schmp.: 207 °C
7) 2-(4-Chlor-phenylsulfonylamino)-N-(4-phenoxy-phenyl)-benzamid
   Schmp.: 143 °C
8) 5-Chlor-2-(4-chlor-phenylsulfonylamino)-N-(3,4-dichlor-phenyl)-benzamid
   Schmp.: 244 °C
9) 5-Chlor-2-(4-chlor-phenylsulfonylamino)-N-(4-brom-phenyl)-benzamid
   Schmp.: 210 °C
10) 5-Chlor-2-(4-chlor-phenylsulfonylamino)-N-(3-chlor-phenyl)-benzamid
   Schmp.: 228 °C
11) 5-Chlor-2-(4-chlor-phenylsulfonylamino)-N-(4-methoxy-phenyl)-benzamid
   Schmp.: 190 °C
12) 5-Chlor-2-(4-chlor-phenylsulfonylamino)-N-(2-naphthyl)-benzamid
   Schmp.: 211 °C
13) 5-Chlor-2-(4-fluor-phenylsulfonylamino)-N-(3,5-dichlor-phenyl)-benzamid
   Schmp.: 250 °C
14) 4-(5-Chlor-2-(4-chlor-phenylsulfonylamino)-benzoylamino)-benzoesäureethylester
   Schmp.: 185°C
15) 5-Chlor-2-(4-chlor-phenylsulfonylamino)-N-(4-(bis-methylsulfonyl-amino)-phenyl)-benzamid
   Schmp.: 235 °C
16) 5-Chlor-2-(4-chlor-phenylsulfonylamino)-N-(4-isopropyl-phenyl)-benzamid
   Schmp.: 188 °C
17) 5-Chlor-2-(3-chlor-4-methoxy-phenylsulfonylamino)-N-(4-fluor-phenyl)-benzamid
   Schmp.: 188 °C
18) 2-Chlor-5-(5-chlor-2-(3,4-dichlor-phenylsulfonylamino)-benzoylamino)-benzoesäureethylester
   Schmp.: 202 °C
19) 5-Chlor-2-(4-chlor-phenylsulfonylamino)-N-(3-chlor-4-(4-chlor-naphthalin-1-yloxy)-phenyl)-benzamid
20) 2-(4-Chlor-phenylsulfonylamino)-5-chlor-N-(4-tert-butyl-phenyl)-benzamid
   Schmp.: 91 °C
21) 5-Chlor-2-(4-chlor-phenylsulfonylamino)-N-(4-(morpholin-4-yl)-phenyl)-benzamid
   Schmp.: 228.5 °C
22) 5-Chlor-2-(4-chlor-phenylsulfonylamino)-N-(4-(phenylamino)-phenyl)-benzamid
   Schmp.: 192.5 C
23) 5-Chlor-2-(4-chlor-phenylsulfonylamino)-N-(4-(benzyl-oxy)-phenyl)-benzamid
   Schmp.: 191 °C
24) 5-Chlor-2-(4-chlor-phenylsulfonylamino)-N-(4-acetyl-phenyl)-benzamid
   Schmp.: 226 °C
25) 2-Phenylsulfonylamino-5-chlor-N-(4-(2-oxo-pyrrolidin-1-yl)-phenyl)-benzamid
   Schmp.: 218 °C
28) 5-Chlor-2-(4-chlor-phenylsulfonylamino)-N-(3,5-bis-trifluormethyl-phenyl)-benzamid
   Schmp.: 182.5 °C
29) 5-Chlor-2-(4-chlor-phenylsulfonylamino)-N-(2-methoxy-5-trifluormethylphenyl)-benzamid
   Schmp.: 164.5 °C
30) 5-Chlor-2-(4-chlor-phenylsulfonylamino)-N-(2-trifluormethyl-phenyl)-benzamid
   Schmp.: 182 °C
31) 5-Chlor-2-(4-chlor-phenylsulfonylamino)-N-(3-methoxy-phenyl)-benzamid
   Schmp.: 163.5 °C
32) 5-Chlor-2-(4-chlor-phenylsulfonylamino)-N-(3,5-dimethoxy-phenyl)-benzamid
   Schmp.: 74.5 °C
33) 5-Chlor-2-(4-chlor-phenylsulfonylamino)-N-(4-(morpholin-4-carbonyl)-phenyl)-benzamid
34) 5-Chlor-2-(4-chlor-phenylsulfonylamino)-N-(4-(2,5-dioxo-pyrrolidin-1-yl)-phenyl)-benzamid
39) 5-Chlor-2-(4-chlor-phenylsulfonylamino)-N-(4-ethoxy-2-nitro-phenyl)-benzamid
40) 5-Chlor-2-(4-chlor-phenylsulfonylamino)-N-(2-methoxy-5-nitro-phenyl)-benzamid
41 ) 5-Chlor-2-(4-chlor-phenylsulfonylamino)-N-(2,5-dimethoxy-4-nitro-phenyl)-benzamid
42) 5-Chlor-2-(4-chlor-phenylsulfonylamino)-N-(2-methoxy-4-nitro-phenyl)-benzamid
43) 5-Chlor-2-(4-chlor-phenylsulfonylamino)-N-(2-cyan-phenyl)-benzamid
44) 5-Chlor-2-(4-chlor-phenylsulfonylamino)-N-(4-cyan-2,3,5,6-tetrafluor-phenyl)-benzamid
45) 5-Chlor-2-(4-chlor-phenylsulfonylamino)-N-(2-(2-hydroxy-ethyl)-phenyl)-benzamid
46) 5-Chlor-2-(4-chlor-phenylsulfonylamino)-N-(3-cyan-phenyl)-benzamid
47) 5-Chlor-2-(4-chlor-phenylsulfonylamino)-N-(4-benzoylamino-5-chlor-2-methyl-phenyl)-benzamid
48) 5-Chlor-2-(4-chlor-phenylsulfonylamino)-N-(3-(1-hydroxy-ethyl)-phenyl)-benzamid
49) 5-Chlor-2-(4-chlor-phenylsulfonylamino)-N-(4-cyan-phenyl)-benzamid
50) N-(2-Benzoyl-4-chlor-phenyl)-5-chlor-2-(4-chlor-phenylsulfonylamino)-benzamid
51 ) 5-Chlor-2-(4-chlor-phenylsulfonylamino)-N-(4-diethylamino-phenyl)-benzamid
52) N-(4-Butoxy-phenyl)-5-chlor-2-(4-chlor-phenylsulfonylamino)-benzamid
53) 5-Chlor-2-(4-chlor-phenylsulfonylamino)-N-(4-hexyloxy-phenyl)-benzamid
54) (4-(5-Chlor-2-(4-chlor-phenylsulfonylamino)-benzoylamino)-benzyl)-phosphonsäurediethylester
55) 5-Chlor-2-(4-chlor-phenylsulfonylamino)-N-(4-pentyl-phenyl)-benzamid
56) 5-Chlor-2-(4-chlor-phenylsulfonylamino)-N-(3,4,5-trimethoxy-phenyl)-benzamid
57) 2-(4-(5-Chlor-2-(4-chlor-phenylsulfonylamino)-benzoylamino)-benzoylamino)-pentandisäurediethylester
59) 4-(5-Chlor-2-(4-chlor-phenylsulfonylamino)-benzoylamino)-benzoesäurebutylester
60) 5-Chlor-2-(4-chlor-phenylsulfonylamino)-N-(2-phenoxy-phenyl)-benzamid
61) 5-Chlor-2-(4-chlor-phenylsulfonylamino)-N-(3-phenoxy-phenyl)-benzamid
62) 5-Chlor-2-(4-chlor-phenylsulfonylamino)-N-(4-hydroxy-biphenyl-3-yl)-benzamid
63) 5-Chlor-2-(4-chlor-phenylsulfonylamino)-N-(4-hydroxy-2-nitro-phenyl)-benzamid
64) 5-Chlor-2-(4-chlor-phenylsulfonylamino)-N-(3-hydroxymethyl-2-methylphenyl)-benzamid
65) 5-Chlor-2-(4-chlor-phenylsulfonylamino)-N-(5-hydroxymethyl-2-methylphenyl)-benzamid
66) 5-Chlor-2-(4-chlor-phenylsulfonylamino)-N-(4-(4-methyl-phenylsulfonylamino)-phenyl)-benzamid
67) 2-(5-Chlor-2-(4-chlor-phenylsulfonylamino)-benzoylamino)-benzoesäure
68) (4-(5-Chlor-2-(4-chlor-phenylsulfonylamino)-benzoylamino)-phenyl)-carbaminsäure-tert-butylester
69) 5-Chlor-2-(4-chlor-phenylsulfonylamino)-N-(3,4,5-trifluor-phenyl)-benzamid
71) 3-(5-Chlor-2-(4-chlor-phenylsulfonylamino)-benzoylamino)-benzoesäure
72) 4-(5-Chlor-2-(4-chlor-phenylsulfonylamino)-benzoylamino)-benzoesäure-(2-diethylaminoethyl)-ester
74) 5-Chlor-2-(4-chlor-phenylsulfonylamino)-N-(2-hydroxymethyl-4-methylphenyl)-benzamid
75) 5-Chlor-2-(4-chlor-phenylsulfonylamino)-N-(2-(pyrrol-1-yl)-phenyl)-benzamid
77) 3-(5-Chlor-2-(4-chlor-phenylsulfonylamino)-benzoylamino)-benzoesäureethylester
78) N-(Benzo[1,3]dioxol-5-yl)-5-chlor-2-(4-chlor-phenylsulfonylamino)-benzamid
79) 5-Chlor-2-(4-chlor-phenylsulfonylamino)-N-(3-chlor-4-cyan-phenyl)-benzamid
80) 2-(5-Chlor-2-(4-chlor-phenylsulfonylamino)-benzoylamino)-4,5-dimethoxybenzoesäuremethylester
81) 5-Chlor-2-(4-chlor-phenylsulfonylamino)-N-(3-nitro-phenyl)-benzamid
82) 3-(5-Chlor-2-(4-chlor-phenylsulfonylamino)-benzoylamino)-benzoesäuremethylester
83) 5-Chlor-2-(4-chlor-phenylsulfonylamino)-N-(2-(morpholin-4-yl)-5-trifluormethyl-phenyl)-benzamid
86) N-(2-(1H-Benzimidazol-2-yl)-phenyl)-5-chlor-2-(4-chlor-phenylsulfonylamino)-benzamid
87) 5-Chlor-2-(4-chlor-phenylsulfonylamino)-N-(2-(N-phenylcarbamoyl)-phenyl)-benzamid
88) N-(3-Benzoyl-phenyl)-5-chlor-2-(4-chlor-phenylsulfonylamino)-benzamid
89) 4-(5-Chlor-2-(4-chlor-phenylsulfonylamino)-benzoylamino)-2-methoxybenzoesäuremethylester
90) 5-Chlor-2-(4-chlor-phenylsulfonylamino)-N-(2-carbamoyl-phenyl)-benzamid
91) 5-Chlor-2-(4-chlor-phenylsulfonylamino)-N-(4-carbamoyl-phenyl)-benzamid
92) 5-Chlor-2-(4-chlor-phenylsulfonylamino)-N-(3-carbamoyl-2-methoxy-phenyl)-benzamid
93) 5-Chlor-2-(4-chlor-phenylsulfonylamino)-N-(3-diethylaminomethyl-4-hydroxyphenyl)-benzamid
94) 5-Chlor-2-(4-chlor-phenylsulfonylamino)-N-(2,5-diethoxy-4-(morpholin-4-yl)-phenyl)-benzamid
95) N-(3-Acetylamino-phenyl)-5-chlor-2-(4-chlor-phenylsulfonylamino)-benzamid
97) 5-Chlor-2-(4-chlor-phenylsulfonylamino)-N-(4-(cyan-phenyl-methyl)-phenyl)-benzamid
98) 5-Chlor-2-(4-chlor-phenylsulfonylamino)-N-(4-(ethyl-(2-hydroxy-ethyl)-amino)-phenyl)-benzamid
99) 5-Chlor-2-(4-chlor-phenylsulfonylamino)-N-(2-(2,4-dichlor-phenoxy)-phenyl)-benzamid
101) N-(4-(5-Chlor-2-(4-chlor-phenylsulfonylamino)-benzoylamino)-phenyl)-oxalsäureamid
102) (4-(5-Chlor-2-(4-chlor-phenylsulfonylamino)-benzoylamino)-benzoylamino)-essigsäure
103) (4-(5-Chlor-2-(4-chlor-phenylsulfonylamino)-benzoylamino)-phenyl)-essigsäure
104) 3-(4-(5-Chlor-2-(4-chlor-phenylsulfonylamino)-benzoylamino)-benzoylamino)-propionsäure
105) (4-(5-Chlor-2-(4-chlor-phenylsulfonylamino)-benzoylamino)-benzyl)-phosphonsäure
106) 4-(4-(5-Chlor-2-(4-chlor-phenylsulfonylamino)-benzoylamino)-phenyl)-buttersäure
107) 2-(4-(5-Chlor-2-(4-chlor-phenylsulfonylamino)-benzoylamino)-benzoylamino)-pentandisäure
108) (3-(5-Chlor-2-(4-chlor-phenylsulfonylamino)-benzoylamino)-phenyl)-essigsäure

### Pharmakologische Untersuchungen

### 1) Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enyzm-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.

Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM MgCl₂, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen Lösungsmittel. Die Prüfsubstanzen wurden in Dimethylsulfoxid (DMSO) gelöst und die Lösung mit DMSO/Wasser verdünnt, so daß die Endkonzentration c an Prüfsubstanz im Testansatz den unten angegebenen Wert hatte. Die DMSO-Konzentration im Testansatz betrug 5 % (v/v). Die Reaktion wurde durch Zugabe der sGC gestartet. Der Reaktionsmix wurde für 15 bis 20 Minuten bei 37 °C inkubiert und dann durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde (berechnet nach der Formel n-fache Stimulierung = [cGMP]_{Prüfsubstanz} / [cGMP]_{Kontrolle}).

Es wurden folgende Ergebnisse erhalten:

| Verbindung des Beispiels Nr. | Konzentration c (µM) | n-fache Stimulation |
|---|---|---|
| 1 | 100 | 3.6 |
| 2 | 100 | 9.2 |
| 3 | 100 | 2 |
| 4 | 25 | 5.5 |
| 5 | 10 | 9.3 |
| 6 | 100 | 4.2 |
| 7 | 100 | 2.8 |
| 8 | 10 | 5.3 |
| 9 | 100 | 1.6 |
| 10 | 100 | 1.7 |
| 11 | 100 | 1.8 |
| 12 | 25 | 2.8 |
| 13 | 100 | 1.8 |
| 14 | 100 | 1.7 |
| 15 | 10 | 9.9 |
| 16 | 100 | 4.1 |
| 17 | 100 | 2 |
| 18 | 100 | 3.2 |
| 19 | 100 | 26.3 |
| 21 | 50 | 8 |
| 23 | 25 | 2.2 |
| 24 | 50 | 2.4 |
| 25 | 50 | 2.4 |
| 33 | 50 | 3.8 |
| 93 | 50 | 1.5 |
| 97 | 12.5 | 4.9 |
| 102 | 50 | 1.7 |
| 108 | 50 | 2.5 |

## Patentansprüche

1. Verwendung einer Verbindung der Formel I, 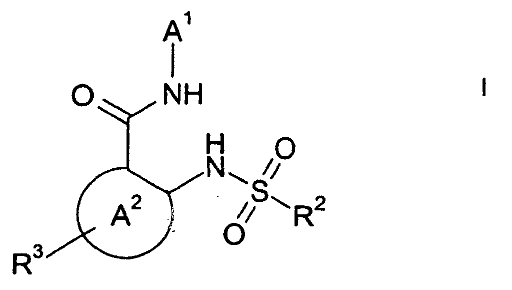 in der
A¹ für einen Rest aus der Reihe Phenyl und Naphthyl steht, die alle durch einen oder mehrere gleiche oder verschiedene Reste R¹ substituiert sein können;
der Ring A², der die Kohlenstoffatome umfaßt, die die Gruppen CO-NH- und NH-SO₂R² tragen, für einen Benzolring steht;
R¹ für Halogen, Aryl, CF₃, NO₂, OH, -O-(C₁-C₇)-Alkyl, -O-(C₂-C₄)-Alkyl-O-(C₁-C₇)-Alkyl, -O-Aryl, (C₁-C₂)-Alkylendioxy, NR⁵R⁶, CN, CO-NR⁵R⁶, COOH, CO-O-(C₁-C₅)-Alkyl, Heterocyclyl, CHO, CO-(C₁-C₁₀)-Alkyl, CO-Aryl oder für (C₁-C₁₀)-Alkyl, das durch einen oder mehrere gleiche oder verschiedene Reste R⁴ substituiert sein kann, steht;
R² für Phenyl, das durch einen oder mehrere gleiche oder verschiedene Substituenten aus der Reihe Halogen, CF₃, (C₁-C₃)-Alkyl, Cyan, Nitro und (C₁-C₃)-Alkoxy substituiert sein kann, steht;
R³ für einen oder mehrere gleiche oder verschiedene Substituenten aus der Reihe Wasserstoff und Halogen steht;
R⁴ für Fluor, OH, -O-(C₁-C₁₀)-Alkyl, -O-(C₁-C₇)-Alkyl-R⁷, -O-Aryl, SH, -S-(C₁-C₁₀)-Alkyl, -S-(C₁-C₇)-Alkyl-R⁷, -S-Aryl, -P(O)(O-(C₁-C₅)-Alkyl)₂, -P(O)(OH)₂, CN, NR⁸R⁹, CO-NH₂, CO-NH-(C₁-C₃)-Alkyl, CO-N((C₁-C₃)-Alkyl)₂, COOH, CO-O-(C₁-C₅)-Alkyl, Heterocyclyl oder Oxo steht;
R⁵ für Wasserstoff, (C₁-C₁₀)-Alkyl, das durch einen oder mehrere gleiche oder verschiedene Substituenten R⁴ und/oder durch Aryl substituiert sein kann, Aryl, Heterocyclyl, CO-(C₁-C₁₀)-Alkyl, CO-Aryl, CO-Heterocyclyl, SO₂-(C₁-C₁₀)-Alkyl, SO₂-Aryl oder SO₂-Heterocyclyl steht;
R⁶ unabhängig von R⁵ eine der für R⁵ angegebenen Bedeutungen hat, oder R⁵ und R⁶ zusammen mit dem Stickstoffatom, an das sie gebunden sind, einen 5-gliedrigen bis 8-gliedrigen gesättigten oder teilweise ungesättigten Ring bilden, der zusätzlich zu dem die Gruppen R⁵ und R⁶ tragenden Stickstoffatom noch ein oder mehrere weitere Ring-Heteroatome aus der Reihe N, O und S enthalten kann und der durch einen oder mehrere gleiche oder verschiedene Substituenten aus der Reihe Fluor, (C₁-C₅)-Alkyl, (C₁-C₃)-Hydroxyalkyl, -(C₁-C₃)-Alkyl-O-(C₁-C₄)-Alkyl, Aryl, CF₃, OH, -O-(C₁-C₇)-Alkyl, -O-Aryl, -O-(C₂-C₄)-Alkyl-O-(C₁-C₇)-Alkyl, (C₂-C₃)-Alkylendioxy, NR⁸R⁹, CN, CO-NH₂, CO-NH-(C₁-C₃)-Alkyl, CO-N((C₁-C₃)-Alkyl)₂, COOH, CO-O-(C₁-C₅)-Alkyl, CHO, CO-(C₁-C₅)-Alkyl, S(O)ₙ-(C₁-C₄)-Alkyl, S(O)ₙ-NH₂, S(O)ₙ-NH-(C₁-C₃)-Alkyl, S(O)ₙ-N((C₁-C₃)-Alkyl)₂, Oxo, -(CH₂)ₘ-NH₂, -(CH₂)ₘ-NH-(C₁-C₄)-Alkyl und -(CH₂)ₘ-N((C₁-C₄)-Alkyl)₂ substituiert sein kann, wobei in dem Substituenten -(CH₂)ₘ-N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁷ für OH, -O-(C₁-C₇)-Alkyl, NH₂, -NH-(C₁-C₄)-Alkyl oder-N((C₁-C₄)-Alkyl)₂ steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁸ für Wasserstoff oder (C₁-C₇)-Alkyl, das durch einen oder mehrere gleiche oder verschiedene Substituenten aus der Reihe OH, -O-(C₁-C₅)-Alkyl, NH₂, -NH-(C₁-C₄)-Alkyl und -N((C₁-C₄)-Alkyl)₂ substituiert sein kann, steht;
R⁹ unabhängig von R⁸ eine der Bedeutungen von R⁸ hat oder für CO-(C₁-C₄)-Alkyl steht;
Aryl für Phenyl, Naphthyl oder Heteroaryl steht, die alle durch einen oder mehrere gleiche oder verschiedene Substituenten aus der Reihe Halogen, (C₁-C₅)-Alkyl, Phenyl, Tolyl, CF₃, NO₂, OH, -O-(C₁-C₅)-Alkyl, -O-(C₂-C₄)-Alkyl-O-(C₁-C₃)-Alkyl, (C₁-C₂)-Alkylendioxy, NH₂, -NH-(C₁-C₃)-Alkyl, -N((C₁-C₃)-Alkyl)₂, NH-CHO, -NH-CO-(C₁-C₅)-Alkyl, CN, CO-NH₂, CO-NH-(C₁-C₃)-Alkyl, CO-N((C₁-C₃)-Alkyl)₂, COOH, CO-O-(C₁-C₅)-Alkyl, Heterocyclyl, CHO, CO-(C₁-C₅)-Alkyl, S(O)ₙ-(C₁-C₄)-Alkyl, S(O)ₙ-Phenyl, S(O)ₙ-Tolyl, S(O)₂-NH₂, S(O)₂-NH-(C₁-C₃)-Alkyl und S(O)₂-N((C₁-C₃)-Alkyl)₂, substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere Ring-Heteroatome aus der Reihe N, O und S enthalten;
Heterocyclyl für den Rest eines monocyclischen oder polycyclischen, 5-gliedrigen bis 11-gliedrigen gesättigten oder teilweise ungesättigten Heterocyclus steht, der ein oder mehrere Ring-Heteroatome aus der Reihe N, O und S enthält und der durch einen oder mehrere gleiche oder verschiedene Substituenten aus der Reihe Fluor, (C₁-C₅)-Alkyl, OH, -O-(C₁-C₅)-Alkyl, -O-(C₂-C₄)-Alkyl-O-(C₁-C₃)-Alkyl, NH₂, -NH-(C₁-C₃)-Alkyl, -N((C₁-C₃)-Alkyl)₂, CN, CO-NH₂, CO-NH-(C₁-C₃)-Alkyl, CO-N((C₁-C₃)-Alkyl)₂, COOH und CO-O-(C₁-C₅)-Alkyl substituiert sein kann;
n für 0, 1 oder 2 steht;
m für 2, 3 oder 4 steht;
in allen ihren stereoisomeren Formen und Mischungen davon in allen Verhältnissen, und/oder ihrer physiologisch verträglichen Salze zur Herstellung eines Medikaments zur Aktivierung der löslichen Guanylatcyclase.

2. Verwendung einer Verbindung der Formel I gemäß Anspruch 1 und/oder ihrer physiologisch verträglichen Salze, worin A¹ für Phenyl, das durch einen oder mehrere gleiche oder verschiedene Reste R¹ substituiert sein kann, steht.

3. Verwendung einer Verbindung der Formel I gemäß Anspruch 1 und/oder 2 und/oder ihrer physiologisch verträglichen Salze, worin R¹ für Halogen, Trifluormethyl, (C₃-C₇)-Alkyl, Carboxymethyl, CONR⁵R⁶, 5-gliedriges bis 7-gliedriges Heterocyclyl, -O-Aryl, -CO-(C₁-C₁₀)-Alkyl, -CO-Aryl, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-Aryl)₂ oder -N(SO₂-(C₁-C₁₀)-Alkyl)₂ steht, wobei alle Alkylreste durch einen oder mehrere gleiche oder verschiedene Reste R⁴ substituiert sein können.

4. Verwendung einer Verbindung der Formel I gemäß einem oder mehreren der Ansprüche 1 bis 3 und/oder ihrer physiologisch verträglichen Salze, worin R¹ für Halogen, Trifluormethyl, -O-Aryl, -NH-CO-(C₁-C₁₀)-Alkyl, -NH-CO-Aryl, -NH-SO₂-(C₁-C₁₀)-Alkyl oder -NH-SO₂-Aryl steht, wobei alle Alkylreste durch einen oder mehrere gleiche oder verschiedene Reste R⁴ substituiert sein können.

5. Verwendung einer Verbindung der Formel I gemäß einem oder mehreren der Ansprüche 1 bis 3 und/oder ihrer physiologisch verträglichen Salze, worin R¹ für CONR⁵R⁶, -CO-(C₁-C₁₀)-Alkyl, -CO-Aryl, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-Aryl)₂ oder -N(SO₂-(C₁-C₁₀)-Alkyl)₂ steht, wobei alle Alkylreste durch einen oder mehrere gleiche oder verschiedene Reste R⁴ substituiert sein können.

6. Verwendung einer Verbindung der Formel I gemäß einem oder mehreren der Ansprüche 1 bis 5 und/oder ihrer physiologisch verträglichen Salze zur Herstellung eines Medikaments zur Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lemfähigkeit oder Gedächtnisleistung.

7. Verbindung der Formel Ik, 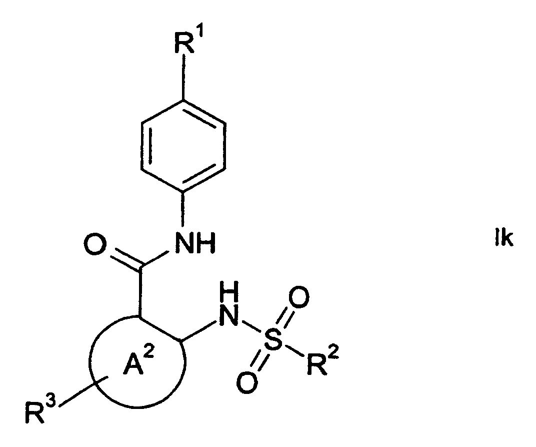 in der
der Ring A², der die Kohlenstoffatome umfaßt, die die Gruppen CO-NH- und NH-SO₂R² tragen, für einen Benzolring steht;
R¹ für einen Substituenten aus der Reihe CO-(C₁-C₁₀)-Alkyl, CO-Aryl, CO-NR⁵R⁶, -NH-CO-(C₁-C₁₀)-Alkyl, -NH-CO-Aryl, -N(CO-(C₁-C₁₀)-Alkyl)₂, -N(CO-Aryl)₂, -NH-SO₂-(C₁-C₁₀)-Alkyl, -NH-SO₂-Aryl, -N(SO₂-(C₁-C₁₀)-Alkyl)₂, und -N(SO₂-Aryl)₂, worin alle Alkylreste durch einen oder mehrere gleiche oder verschiedene Reste R⁴ substituiert sein können, steht;
R² für Phenyl, das durch einen oder mehrere gleiche oder verschiedene Substituenten aus der Reihe Halogen, CF₃, (C₁-C₃)-Alkyl, Cyan, Nitro und (C₁-C₃)-Alkoxy substituiert sein kann, steht;
R³ für einen oder mehrere gleiche oder verschiedene Substituenten aus der Reihe Wasserstoff und Halogen steht;
R⁴ für Fluor, OH, -O-(C₁-C₁₀)-Alkyl, -O-(C₁-C₇)-Alkyl-R⁷, -O-Aryl, SH, -S-(C₁-C₁₀)-Alkyl, -S-(C₁-C₇)-Alkyl-R⁷, -S-Aryl, -P(O)(O-(C₁-C₅)-Alkyl)₂, -P(O)(OH)₂, CN, NR⁸R⁹, CO-NH₂, CO-NH-(C₁-C₃)-Alkyl, CO-N((C₁-C₃)-Alkyl)₂, COOH, CO-O-(C₁-C₅)-Alkyl, Heterocyclyl oder Oxo steht;
R⁵ für Wasserstoff, (C₁-C₁₀)-Alkyl, das durch einen oder mehrere gleiche oder verschiedene Substituenten R⁴ und/oder durch Aryl substituiert sein kann, Aryl, Heterocyclyl, CO-(C₁-C₁₀)-Alkyl, CO-Aryl, CO-Heterocyclyl, SO₂-(C₁-C₁₀)-Alkyl, SO₂-Aryl oder SO₂-Heterocyclyl steht;
R⁶ unabhängig von R⁵ eine der für R⁵ angegebenen Bedeutungen hat, oder R⁵ und R⁶ zusammen mit dem Stickstoffatom, an das sie gebunden sind, einen 5-gliedrigen bis 8-gliedrigen gesättigten oder teilweise ungesättigten Ring bilden, der zusätzlich zu dem die Gruppen R⁵ und R⁶ tragenden Stickstoffatom noch ein oder mehrere weitere Ring-Heteroatome aus der Reihe N, O und S enthalten kann und der durch einen oder mehrere gleiche oder verschiedene Substituenten aus der Reihe Fluor, (C₁-C₅)-Alkyl, (C₁-C₃)-Hydroxyalkyl, -(C₁-C₃)-Alkyl-O-(C₁-C₄)-Alkyl, Aryl, CF₃, OH, -O-(C₁-C₇)-Alkyl, -O-Aryl, -O-(C₂-C₄)-Alkyl-O-(C₁-C₇)-Alkyl, (C₂-C₃)-Alkylendioxy, NR⁸R⁹, CN, CO-NH₂, CO-NH-(C₁-C₃)-Alkyl, CO-N((C₁-C₃)-Alkyl)₂, COOH, CO-O-(C₁-C₅)-Alkyl, CHO, CO-(C₁-C₅)-Alkyl, S(O)ₙ-(C₁-C₄)-Alkyl, S(O)ₙ-NH₂, S(O)ₙ-NH-(C₁-C₃)-Alkyl, S(O)ₙ-N((C₁-C₃)-Alkyl)₂, Oxo, -(CH₂)ₘ-NH₂, -(CH₂)ₘ-NH-(C₁-C₄)-Alkyl und -(CH₂)ₘ-N((C₁-C₄)-Alkyl)₂ substituiert sein kann, wobei in dem Substituenten -(CH₂)ₘ-N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁷ für OH, -O-(C₁-C₇)-Alkyl, NH₂, -NH-(C₁-C₄)-Alkyl oder -N((C₁-C₄)-Alkyl)₂ steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁸ für Wasserstoff oder (C₁-C₇)-Alkyl, das durch einen oder mehrere gleiche oder verschiedene Substituenten aus der Reihe OH, -O-(C₁-C₅)-Alkyl, NH₂, -NH-(C₁-C₄)-Alkyl und -N((C₁-C₄)-Alkyl)₂ substituiert sein kann, steht, wobei in dem Substituenten N((C₁-C₄)-Alkyl)₂ die beiden Alkylgruppen durch eine Einfachbindung verknüpft sein können und dann zusammen mit dem sie tragenden Stickstoffatom einen 5-gliedrigen bis 7-gliedrigen Ring bilden, der neben dem Stickstoffatom und den Kohlenstoffatomen zusätzlich auch noch ein Sauerstoffatom, ein Schwefelatom oder eine Gruppe NR⁵ als Ringglied enthalten kann;
R⁹ unabhängig von R⁸ eine der Bedeutungen von R⁸ hat oder für CO-(C₁-C₄)-Alkyl steht;
Aryl für Phenyl, Naphthyl oder Heteroaryl steht, die alle durch einen oder mehrere gleiche oder verschiedene Substituenten aus der Reihe Halogen, (C₁-C₅)-Alkyl, Phenyl, Tolyl, CF₃, NO₂, OH, -O-(C₁-C₅)-Alkyl, -O-(C₂-C₄)-Alkyl-O-(C₁-C₃)-Alkyl, (C₁-C₂)-Alkylendioxy, NH₂, -NH-(C₁-C₃)-Alkyl, -N((C₁-C₃)-Alkyl)₂, NH-CHO, -NH-CO-(C₁-C₅)-Alkyl, CN, CO-NH₂, CO-NH-(C₁-C₃)-Alkyl, CO-N((C₁-C₃)-Alkyl)₂, COOH, CO-O-(C₁-C₅)-Alkyl, Heterocyclyl, CHO, CO-(C₁-C₅)-Alkyl, S(O)ₙ-(C₁-C₄)-Alkyl, S(O)ₙ-Phenyl, S(O)ₙ-Tolyl, S(O)₂-NH₂, S(O)₂-NH-(C₁-C₃)-Alkyl und S(O)₂-N((C₁-C₃)-Alkyl)₂, substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere Ring-Heteroatome aus der Reihe N, O und S enthalten;
Heterocyclyl für den Rest eines monocyclischen oder polycyclischen, 5-gliedrigen bis 11-gliedrigen gesättigten oder teilweise ungesättigten Heterocyclus steht, der ein oder mehrere Ring-Heteroatome aus der Reihe N, O und S enthält und der durch einen oder mehrere gleiche oder verschiedene Substituenten aus der Reihe Fluor, (C₁-C₅)-Alkyl, OH, -O-(C₁-C₅)-Alkyl, -O-(C₂-C₄)-Alkyl-O-(C₁-C₃)-Alkyl, NH₂, -NH-(C₁-C₃)-Alkyl, -N((C₁-C₃)-Alkyl)₂, CN, CO-NH₂, CO-NH-(C₁-C₃)-Alkyl, CO-N((C₁-C₃)-Alkyl)₂, COOH und CO-O-(C₁-C₅)-Alkyl substituiert sein kann;
n für 0, 1 oder 2 steht;
m für 2, 3 oder 4 steht;
in allen ihren stereoisomeren Formen und Mischungen davon in allen Verhältnissen, und ihre physiologisch verträglichen Salze.

8. Verbindung der Formel Ik gemäß Anspruch 7 und/oder ihre physiologisch verträglichen Salze zur Anwendung als Arzneimittel.

9. Pharmazeutisches Präparat, **dadurch gekennzeichnet, daß** es eine oder mehrere Verbindungen der Formel Ik gemäß Anspruch 7 und/oder ihre physiologisch verträglichen Salze neben pharmazeutisch einwandfreien Trägerstoffen und/oder Zusatzstoffen enthält.

## Claims

1. The use of a compound of the formula I 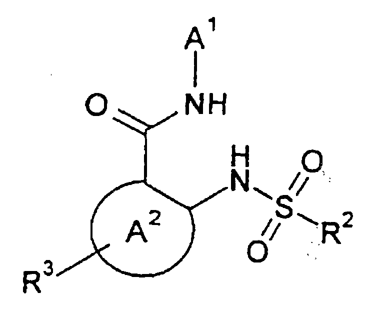 in which
A¹ is a radical from the group consisting of phenyl and naphthyl which can all be substituted by one or more identical or different radicals R¹;
the ring A², which includes the carbon atoms which carry the groups CO-NH- and NH-SO₂R², is a benzene ring;
R¹ is halogen, aryl, CF₃, NO₂, OH, -O-(C₁-C₇)-alkyl, -O-(C₂-C₄)-alkyl-O-(C₁-C₇)-alkyl, -O-aryl, (C₁-C₂)-alkylenedioxy, NR⁵R⁶, CN, CO-NR⁵R⁶, COOH, CO-O-(C₁-C₅)-alkyl, heterocyclyl, CHO, CO-(C₁-C₁₀)-alkyl, CO-aryl or (C₁-C₁₀)-alkyl which can be substituted by one or more identical or different radicals R⁴;
R² is phenyl, which can be substituted by one or more identical or different substituents from the group consisting of halogen, CF₃, (C₁-C₃)-alkyl, cyano, nitro and (C₁-C₃)-alkoxy;
R³ is one or more identical or different substituents from the group consisting of hydrogen and halogen;
R⁴ is fluorine, OH, -O-(C₁-C₁₀)-alkyl, -O-(C₁-C₇)-alkyl-R⁷, -O-aryl, SH, -S-(C₁-C₁₀)-alkyl, -S-(C₁-C₇)-alkyl-R⁷, -S-aryl, -P(O)(O-(C₁-C₅)-alkyl)₂, -P(O)(OH)₂, CN, NR⁸R⁹, CO-NH₂, CO-NH-(C₁-C₃)-alkyl, CO-N((C₁-C₃)-alkyl)₂, COOH, CO-O-(C₁-C₅)-alkyl, heterocyclyl or oxo;
R⁵ is hydrogen, (C₁-C₁₀)-alkyl which can be substituted by one or more identical or different substituents R⁴ and/or by aryl, or is aryl, heterocyclyl, CO-(C₁-C₁₀)-alkyl, CO-aryl, CO-heterocyclyl, SO₂-(C₁-C₁₀)-alkyl, SO₂-aryl or SO₂-heterocyclyl;
R⁶, independently of R⁵, has one of the meanings indicated for R⁵, or R⁵ and R⁶, together with the nitrogen atom to which they are bonded, form a 5-membered to 8-membered saturated or partially unsaturated ring which, in addition to the nitrogen atom carrying the groups R⁵ and R⁶, can also contain one or more further ring heteroatoms from the group consisting of N, O and S and which can be substituted by one or more identical or different substituents from the group consisting of fluorine, (C₁-C₅)-alkyl, (C₁-C₃)-hydroxyalkyl, -(C₁-C₃)-alkyl-O-(C₁-C₄)-alkyl, -aryl, CF₃, OH, -O-(C₁-C₇)-alkyl, -O-aryl, -O-(C₂-C₄)-alkyl-O-(C₁-C₇)-alkyl, (C₂-C₃)-alkylenedioxy, NR⁸R⁹, CN, CO-NH₂, CO-NH-(C₁-C₃)-alkyl, CO-N((C₁-C₃)-alkyl)₂, COOH, CO-O-(C₁-C₅)-alkyl, CHO, CO-(C₁-C₅)-alkyl, S(O)ₙ-(C₁-C₄)-alkyl, S(O)ₙ-NH₂, S(O)ₙ-NH-(C₁-C₃)-alkyl, S(O)ₙ-N((C₁-C₃)-alkyl)₂, oxo, -(CH₂)ₘ-NH₂, -(CH₂)ₘ-NH-(C₁-C₄)-alkyl and -(CH₂)ₘ-N((C₁-C₄)-alkyl)₂, where in the substituent -(CH₂)ₘ-N((C₁-C₄)-alkyl)₂ the two alkyl groups can be linked by a single bond and then, together with the nitrogen atom carrying them, form a 5-membered to 7-membered ring which, besides the nitrogen atom and the carbon atoms, can additionally also contain an oxygen atom, a sulfur atom or a group NR⁵ as a ring member;
R⁷ is OH, -O-(C₁-C₇)-alkyl, NH₂, -NH-(C₁-C₄)-alkyl or -N((C₁-C₄)-alkyl)₂, where in the substituent N((C₁-C₄)-alkyl)₂ the two alkyl groups can be linked by a single bond and then, together with the nitrogen atom carrying them, form a 5-membered to 7-membered ring which, besides the nitrogen atom and the carbon atoms can additionally also contain an oxygen atom, a sulfur atom or a group NR⁵ as a ring member;
R⁸ is hydrogen or (C₁-C₇)-alkyl which can be substituted by one or more identical or different substituents from the group consisting of OH, -O-(C₁-C₅)-alkyl, NH₂, -NH-(C₁-C₄)-alkyl and -N((C₁-C₄)-alkyl)₂;
R⁹, independently of R⁸, has one of the meanings of R⁸ or is CO-(C₁-C₄)-alkyl;
aryl is phenyl, naphthyl or heteroaryl, which can all be substituted by one or more identical or different substituents from the group consisting of halogen, (C₁-C₅)-alkyl, phenyl, tolyl, CF₃, NO₂, OH, -O-(C₁-C₅)-alkyl, -O-(C₂-C₄)-alkyl-O-(C₁-C₃)-alkyl, (C₁-C₂)-alkylenedioxy, NH₂, -NH-(C₁-C₃)-alkyl, -N((C₁-C₃)-alkyl)₂, NH-CHO, -NH-CO-(C₁-C₅)-alkyl, CN, CO-NH₂, CO-NH-(C₁-C₃)-alkyl, CO-N((C₁-C₃)-alkyl)₂, COOH, CO-O-(C₁-C₅)-alkyl, heterocyclyl, CHO, CO-(C₁-C₅)-alkyl, S(O)ₙ-(C₁-C₄)-alkyl, S(O)ₙ-phenyl, S(O)ₙ-tolyl, S(O)₂-NH₂, S(O)₂-NH-(C₁-C₃)-alkyl and S(O)₂-N((C₁-C₃)-alkyl)₂;
heteroaryl is the radical of a monocyclic 5-membered or 6-membered aromatic heterocycle or of a bicyclic 8-membered to 10-membered aromatic heterocycle, each of which contain one or more ring heteroatoms from the group consisting of N, O and S;
heterocyclyl is the radical of a monocyclic or polycyclic, 5-membered to 11-membered saturated or partially unsaturated heterocycle which contains one or more ring heteroatoms from the group consisting of N, O and S and which can be substituted by one or more identical or different substituents from the group consisting of fluorine, (C₁-C₅)-alkyl, OH, -O-(C₁-C₅)-alkyl, -O-(C₂-C₄)-alkyl-O-(C₁-C₃)-alkyl, NH₂, -NH-(C₁-C₃)-alkyl, -N((C₁-C₃)-alkyl)₂, CN, CO-NH₂, CO-NH-(C₁-C₃)-alkyl, CO-N((C₁-C₃)-alkyl)₂, COOH and CO-O-(C₁-C₅)-alkyl;
n is 0, 1 or 2;
m is 2, 3 or 4;
in all its stereoisomeric forms and mixtures thereof in all ratios, and/or their physiologically tolerable salts for the production of a medicament for the activation of soluble guanylate cyclase.

2. The use of a compound of the formula I as claimed in claim 1 and/or its physiologically tolerable salts, in which A¹ is phenyl, which can be substituted by one or more identical or different radicals R¹.

3. The use of a compound of the formula I as claimed in claim 1 and/or 2 and/or its physiologically tolerable salts, in which R ¹ is halogen, trifluoromethyl, (C₃-C₇)-alkyl, carboxymethyl, GONR⁵R⁶, 5-membered to 7-membered heterocyclyl, -O-aryl, -CO-(C₁-C₁₀)-alkyl, -CO-aryl, -NH-CO-(C₁-C₁₀)-alkyl, -NH-CO-aryl, -N(CO-(C₁-C₁₀)-alkyl)₂, -N(CO-aryl)₂, -NH-SO₂-(C₁-C₁₀)-alkyl, -NH-SO₂-aryl, -N(SO₂-aryl)₂ or -N(SO₂-(C₁-C₁₀)-alkyl)₂, where all alkyl radicals can be substituted by one or more identical or different radicals R⁴.

4. The use of a compound of the formula I as claimed in one or more of claims 1 to 3 and/or its physiologically tolerable salts, in which R¹ is halogen, trifluoromethyl, -O-aryl, -NH-CO-(C₁-C₁₀)-alkyl, -NH-CO-aryl, -NH-SO₂-(C₁-C₁₀)-alkyl or -NH-SO₂-aryl, where all alkyl radicals can be substituted by one or more identical or different radicals R⁴.

5. The use of a compound of the formula I as claimed in one or more of claims 1 to 3 and/or its physiologically tolerable salts, in which R¹ is CONR⁵R⁶, -CO-(C₁-C₁₀)-alkyl, -CO-aryl, -NH-CO-(C₁-C₁₀)-alkyl, -NH-CO-aryl, -N(CO-(C₁-C₁₀)-alkyl)₂, -N(CO-aryl)₂, -NH-SO₂-(C₁-C₁₀)-alkyl, -NH-SO₂-aryl, -N(SO₂-aryl)₂ or -N(SO₂-(C₁-C₁₀)-alkyl)₂, where all alkyl radicals can be substituted by one or more identical or different radicals R⁴.

6. The use of a compound of the formula I as claimed in one or more of claims 1 to 5 and/or its physiologically tolerable salts for the production of a medicament for the therapy or prophylaxis of cardiovascular disorders, endothelial dysfunction, diastolic dysfunction, atherosclerosis, high blood pressure, angina pectoris, thromboses, restenoses, myocardial infarct, strokes, cardiac insufficiency, pulmonary hypertension, erectile dysfunction, bronchial asthma, chronic renal insufficiency, diabetes or liver cirrhosis or for improving a restricted learning capacity or memory power.

7. A compound of the formula Ik 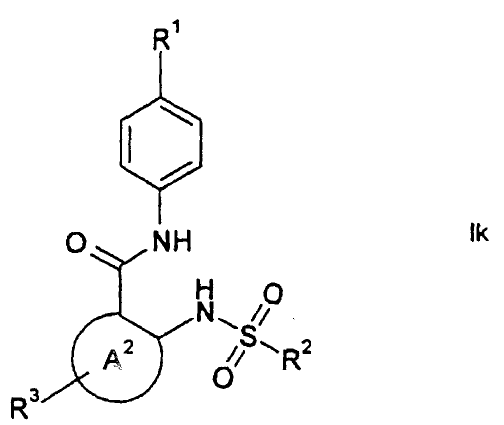 in which
the ring A², which includes the carbon atoms which carry the groups CO-NH- and NH-SO₂R², is a benzene ring;
R¹ is a substituent from the group consisting of CO-(C₁-C₁₀)-alkyl, CO-aryl, CO-NR⁵R⁶, -NH-CO-(C₁-C₁₀)-alkyl, -NH-CO-aryl, -N(CO-(C₁-C₁₀)-alkyl)₂, -N(CO-aryl)₂, -NH-SO₂-(C₁-C₁₀)-alkyl, -NH-SO₂-aryl, -N(SO₂-(C₁-C₁₀)-alkyl)₂ and -N(SO₂-aryl)₂, where all alkyl radicals can be substituted by one or more identical or different radicals R⁴;
R² is phenyl, which can be substituted by one or more identical of different substituents from the group consisting of halogen, CF₃, (C₁-C₃)-alkyl, cyano, nitro and (C₁-C₃)-alkoxy;
R³ is one or more identical or different substituents from the group consisting of hydrogen and halogen;
R⁴ is fluorine, OH, -O-(C₁-C₁₀)-alkyl, -O-(C₁-C₇)-alkyl-R⁷, -O-aryl, SH, -S-(C₁-C₁₀)-alkyl, -S-(C₁-C₇)-alkyl-R⁷, -S-aryl, -P(O)(O-(C₁-C₅)-alkyl)₂, -P(O)(OH)₂, CN, NR⁸R⁹, CO-NH₂, CO-NH-(C₁-C₃)-alkyl, CO-N((C₁-C₃)-alkyl)₂, COOH, CO-O-(C₁-C₅)-alkyl, heterocyclyl or oxo;
R⁵ is hydrogen, (C₁-C₁₀)-alkyl which can be substituted by one or more identical or different substituents R⁴ and/or by aryl, or is aryl, heterocyclyl, CO-(C₁-C₁₀)-alkyl, CO-aryl, CO-heterocyclyl, SO₂-(C₁-C₁₀)-alkyl, SO₂-aryl or SO₂-heterocyclyl;
R⁶, independently of R⁵, has one of the meanings indicated for R⁵, or R⁵ and R⁶, together with the nitrogen atom to which they are bonded, form a 5-membered to 8-membered saturated or partially unsaturated ring, which in addition to the nitrogen atom carrying the groups R⁵ and R⁶ can also contain one or more further ring heteroatoms from the group consisting of N, O and S and which can be substituted by one or more identical or different substituents from the group consisting of fluorine, (C₁-C₅)-alkyl, (C₁-C₃)-hydroxyalkyl, -(C₁-C₃)-alkyl-O-(C₁-C₄)-alkyl, aryl, CF₃, OH, -O-(C₁-C₇)-alkyl, -O-aryl, -O-(C₂-C₄)-alkyl-O-(C₁-C₇)-alkyl, (C₂-C₃)-alkylenedioxy, NR⁸R⁹, CN, CO-NH₂, CO-NH-(C₁-C₃)-alkyl, CO-N((C₁-C₃)-alkyl)₂, COOH, CO-O-(C₁-C₅)-alkyl, CHO, CO-(C₁-C₅)-alkyl, S(O)ₙ-(C₁-C₄)-alkyl, S(O)ₙ-NH₂, S(O)ₙ-NH-(C₁-C₃)-alkyl, S(O)ₙ-N((C₁-C₃)-alkyl)₂, oxo, -(CH₂)ₘ-NH₂, -(CH₂)ₘ-NH-(C₁-C₄)-alkyl and -(CH₂)ₘ-N((C₁-C₄)-alkyl)₂, where in the substituent -(CH₂)ₘ-N((C₁-C₄)-alkyl)₂ the two alkyl groups can be linked by a single bond and then together with the nitrogen atom carrying them form a 5-membered to 7-membered ring which besides the nitrogen atom and the carbon atoms can additionally also contain an oxygen atom, a sulfur atom or a group NR⁵ as a ring member;
R⁷ is OH, -O-(C₁-C₇)-alkyl, NH₂, -NH-(C₁-C₄)-alkyl or -N((C₁-C₄)-alkyl)₂, where in the substituent N((C₁-C₄)-alkyl)₂ the two alkyl groups can be linked by a single bond and then together with the nitrogen atom carrying them form a 5-membered to 7-membered ring which besides the nitrogen atom and the carbon atoms can additionally also contain an oxygen atom, a sulfur atom or a group NR⁵ as a ring member;
R⁸ is hydrogen or (C₁-C₇)-alkyl which can be substituted by one or more identical or different substituents from the group consisting of OH, -O-(C₁-C₅)-alkyl, NH₂, -NH-(C₁-C₄)-alkyl and -N((C₁-C₄)-alkyl)₂, where in the substituent N((C₁-C₄)-alkyl)₂ the two alkyl groups can be linked by a single bond and then together with the nitrogen atom carrying them form a 5-membered to 7-membered ring which besides the nitrogen atom and the carbon atoms can additionally also contain an oxygen atom, a sulfur atom or a group NR⁵ as a ring member;
R⁹, independently of R⁸, has one of the meanings of R⁸ or is CO-(C₁-C₄)-alkyl;
aryl is phenyl, naphthyl or heteroaryl, which can all be substituted by one or more identical or different substituents from the group consisting of halogen, (C₁-C₅)-alkyl, phenyl, tolyl, CF₃, NO₂, OH, -O-(C₁-C₅)-alkyl, -O-(C₂-C₄)-alkyl-O-(C₁-C₃)-alkyl, (C₁-C₂)-alkylenedioxy, NH₂, -NH-(C₁-C₃)-alkyl, -N((C₁-C₃)-alkyl)₂, NH-CHO, -NH-CO-(C₁-C₅)-alkyl, CN, CO-NH₂, CO-NH-(C₁-C₃)-alkyl, CO-N((C₁-C₃)-alkyl)₂, COOH, CO-O-(C₁-C₅)-alkyl, heterocyclyl, CHO, CO-(C₁-C₅)-alkyl, S(O)ₙ-(C₁-C₄)-alkyl, S(O)ₙ-phenyl, S(O)ₙ-tolyl, S(O)₂-NH₂, S(O)₂-NH-(C₁-C₃)-alkyl and S(O)₂-N((C₁-C₃)-alkyl)₂;
heteroaryl is the radical of a monocyclic 5-membered or 6-membered aromatic heterocycle or of a bicyclic 8-membered to 10-membered aromatic heterocycle which in each case contain one or more ring heteroatoms from the group consisting of N, O and S;
heterocyclyl is the radical of a monocyclic or polycyclic, 5-membered to 11-membered saturated or partially unsaturated heterocycle which contains one or more ring heteroatoms from the group consisting of N, O and S and which can be substituted by one or more identical or different substituents from the group consisting of fluorine, (C₁-C₅)-alkyl, OH, -O-(C₁-C₅)-alkyl, -O-(C₂-C₄)-alkyl-O-(C₁-C₃)-alkyl, NH₂, -NH-(C₁-C₃)-alkyl, -N((C₁-C₃)-alkyl)₂, CN, CO-NH₂, CO-NH-(C₁-C₃)-alkyl, CO-N((C₁-C₃)-alkyl)₂, COOH and CO-O-(C₁-C₅)-alkyl;
n is 0, 1 or 2;
m is 2, 3 or 4;
in all its stereoisomeric forms and mixtures thereof in all ratios, and its physiologically tolerable salts.

8. A compound of the formula Ik as claimed in claim 7 and/or its physiologically tolerable salts for use as a pharmaceutical.

9. A pharmaceutical preparation which contains one or more compounds of the formula Ik as claimed in claim 7 and/or their physiologically tolerable salts in addition to pharmaceutically innocuous vehicles and/or additives.

## Revendications

1. Utilisation d'un composé de formule I 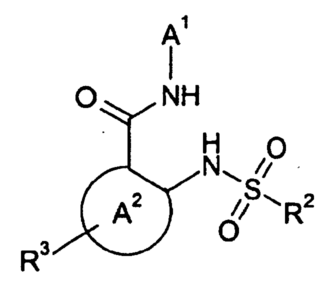 dans laquelle
A¹ représente un radical de la série phényle et naphtyle, qui peuvent tous être substitués par un ou plusieurs radicaux R¹ identiques ou différents ; le cycle A², qui comprend les atomes de carbone qui portent les groupes CO-NH- et NH-SO₂R², représente un cycle benzène ;
R¹ représente halogène, aryle, CF₃, NO₂, OH, -O-alkyle en C₁ à C₇, -O-(alkyle en C₂ à C₄)-O-(alkyle en C₁ à C₇), -O-aryle, (alkylène en C₁ à C₂)dioxy, NR⁵R⁶, CN, CO-NR⁵R⁶, COOH, CO-O-(alkyle en C₁ à C₅), hétérocyclyle, CHO, CO-(alkyle en C₁ à C₁₀), CO-aryle ou alkyle en C₁ à C₁₀, qui peut être substitué par un ou plusieurs radicaux R⁴ identiques ou différents ;
R² représente phényle, qui peut être substitué par un ou plusieurs substituants identiques ou différents de la série halogène, CF₃, alkyle en C₁ à C₃, cyano, nitro et alcoxy en C₁ à C₃ ;
R³ représente un ou plusieurs substituants identiques ou différents de la série hydrogène et halogène ;
R⁴ représente fluor, OH, -O-alkyle en C₁ à C₁₀, -O-(alkyle en C₁ à C₇)-R⁷, -O-aryle, SH, -S-alkyle en C₁ à C₁₀, -S-(alkyle en C₁ à C₇)-R⁷, -S-aryle, -P(O)(O-alkyle en C₁ à C₅)₂, -P(O) (OH)₂, CN, NR⁸R⁹, CO-NH₂, CO-NH-(alkyle en C₁ à C₃), CO-N(alkyle en C₁ à C₃)₂, COOH, CO-O-(alkyle en C₁ à C₅), hétérocyclyle ou oxo;
R⁵ représente hydrogène, alkyle en C₁ à C₁₀, qui peut être substitué par un ou plusieurs substituants R⁴ identiques ou différents et/ou par aryle, aryle, hétérocyclyle, CO-(alkyle en C₁ à C₁₀), CO-aryle, CO-hétérocyclyle, SO₂-alkyle en C₁ à C₁₀, SO₂-aryle ou SO₂-hétérocyclyle ;
R⁶ présente, indépendamment de R⁵, une des significations indiquées pour R⁵, ou R⁵ et R⁶ représentent ensemble avec l'atome d'azote, auquel ils sont liés, un cycle à 5 jusqu'à 8 chaînons, saturé ou partiellement insaturé, qui peut contenir, outre l'atome d'azote qui porte les groupes R⁵ et R⁶, encore un ou plusieurs autres hétéroatomes de cycle de la série N, O et S et qui peut être substitué par un ou plusieurs substituants identiques ou différents de la série fluor, alkyle en C₁ à C₅, hydroxyalkyle en C₁ à C₃, -(alkyle en C₁ à C₃)-O-(alkyle en C₁ à C₄), aryle, CF₃, OH, -O-alkyle en C₁ à C₇, -O-aryle, -O-(alkyle en C₂ à C₄)-O-(alkyle en C₁ à C₇), (alkylène en C₂ à C₃)dioxy, NR⁸R⁹, CN, CO-NH₂, CO-NH-(alkyle en C₁ à C₃), CO-N (alkyle en C₁ à C₃)₂, COOH, CO-O-(alkyle en C₁ à C₅), CHO, CO-(alkyle en C₁ à C₅), S(O)ₙ-alkyle en C₁ à C₄, S(O)ₙ-NH₂, S(O)ₙ-NH-alkyle en C₁ à C₃, S(O)ₙ-N(alkyle en C₁ à C₃)₂, oxo, -(CH₂)ₘ-NH₂, -(CH₂)ₘ-NH-(alkyle en C₁ à C₄) et -(CH₂)ₘ-N(alkyle en C₁ à C₄)₂, où, dans le substituant -(CH₂)ₘ-N(alkyle en C₁ à C₄)₂ les deux groupes alkyle peuvent être liés par une simple liaison et forment alors avec l'atome d'azote qui les porte un cycle à 5 jusqu'à 7 chaînons, qui peut encore contenir, outre l'atome d'azote et les atomes de carbone, un atome d'oxygène, de soufre ou un groupe NR⁵ supplémentaire comme chaînon du cycle ;
R⁷ représente OH, -O-alkyle en C₁ à C₇, NH₂, -NH-alkyle en C₁ à C₄ ou -N(alkyle en C₁ à C₄)₂ où dans le substituant N (alkyle en C₁ à C₄)₂ les deux groupes alkyle peuvent être liés par une simple liaison et forment alors avec l'atome d'azote qui les porte un cycle à 5 jusqu'à 7 chaînons, qui peut encore contenir, outre l'atome d'azote et les atomes de carbone, un atome d'oxygène, de soufre ou un groupe NR⁵ supplémentaire comme chaînon de cycle ;
R⁸ représente hydrogène ou alkyle en C₁ à C₇, qui peut être substitué par un ou plusieurs substituants identiques ou différents de la série OH, -O-alkyle en C₁ à C₅, NH₂, -NH-alkyle en C₁ à C₄ et -N(alkyle en C₁ à C₄)₂ ;
R⁹ présente, indépendamment de R⁸, une des significations de R⁸ ou CO-(alkyle en C₁ à C₄) ; aryle représente phényle, naphtyle ou hétéroaryle, qui peuvent tous être substitués par un ou plusieurs substituants identiques ou différents de la série halogène, alkyle en C₁ à C₅, phényle, tolyle, CF₃, NO₂, OH, -O-alkyle en C₁ à C₅, -O-(alkyle en C₂ à C₄) -O- (alkyle en C₁ à C₃), (alkylène en C₁ à C₂)dioxy, NH₂, -NH-alkyle en C₁ à C₃, -N(alkyle en C₁ à C₃)₂, NH-CHO, -NH-CO-(alkyle en C₁ à C₅), CN, CO-NH₂, CO-NH-(alkyle en C₁ à C₃), CO-N(alkyle en C₁ à C₃)₂, COOH, CO-O-(alkyle en C₁ à C₅), hétérocyclyle, CHO, CO-(alkyle en C₁ à C₅), S(O)ₙ-alkyle en C₁ à C₄, S(O)ₙ-phényle, S(O)ₙ-tolyle, S(O)₂-NH₂, S(O)₂-NH-alkyle en C₁ à C₃ et S(O)₂-N(alkyle en C₁ à C₃)₂ ;
hétéroaryle représente le radical d'un hétérocycle monocyclique aromatique à 5 ou 6 chaînons ou un hétérocycle bicyclique aromatique à 8 jusqu'à 10 chaînons, qui contiennent à chaque fois un ou plusieurs hétéroatomes de cycle de la série N, O et S ;
hétérocyclyle représente le radical d'un hétérocycle monocyclique ou polycyclique, à 5 jusqu'à 11 chaînons, saturé ou partiellement insaturé, qui contient un ou plusieurs hétéroatomes de cycle de la série N, O et S et qui peut être substitué par un ou plusieurs substituants identiques ou différents de la série fluor, alkyle en C₁ à C₅, OH, -O-alkyle en C₁ à C₅, -O-(alkyle en C₂ à C₄)-O-(alkyle en C₁ à C₃), NH₂; -NH-alkyle en C₁ à C₃, -N(alkyle en C₁ à C₃)₂, CN, CO-NH₂, CO-NH-(alkyle en C₁ à C₃), CO-N (alkyle en C₁ à C₃)₂, COOH et CO-O-(alkyle en C₁ à C₅) ;
n vaut 0, 1 ou 2 ;
m vaut 2, 3 ou 4 ;
dans toutes ses formes stereoisomères et tous les mélanges de celles-ci dans tous les rapports et/ou ses sels physiologiquement acceptables pour la préparation d'un médicament destiné à activer la guanylate-cyclase soluble.

2. Utilisation d'un composé de formule I selon la revendication 1 et/ou ses sels physiologiquement acceptables, dans laquelle A¹ représente phényle, qui peut être substitué par un ou plusieurs radicaux R¹ identiques ou différents.

3. Utilisation d'un composé de formule I selon la revendication 1 et/ou 2 et/ou ses sels physiologiquement acceptables, dans laquelle R¹ représente halogène, trifluorométhyle, alkyle en C₃ à C₇, carboxyméthyle, CONR⁵R⁶, hétérocyclyle à 5 jusqu'à 7 chaînons, -O-aryle, -CO- (alkyle en C₁ à C₁₀), -CO-aryle, -NH-CO-(alkyle en C₁ à C₁₀), -NH-CO-aryle, -N(CO-alkyle en C₁ à C₁₀)₂, -N(CO-aryle)₂, -NH-SO₂-alkyle en C₁ à C₁₀, -NH-SO₂-aryle, -N(SO₂-aryle)₂ ou -N(SO₂-alkyle en C₁ à C₁₀)₂, tous les radicaux alkyle pouvant être substitués par un ou plusieurs radicaux R⁴ identiques ou différents.

4. Utilisation d'un composé de formule I selon l'une ou plusieurs des revendications 1 à 3 et/ou ses sels physiologiquement acceptables, dans laquelle R¹ représente halogène, trifluorométhyle, -O-aryle, -NH-CO- (alkyle en C₁ à C₁₀), -NH-CO-aryle, -NH-SO₂-alkyle en C₁ à C₁₀ ou -NH-SO₂-aryle, tous les radicaux alkyle pouvant être substitués par un ou plusieurs radicaux R⁴ identiques ou différents.

5. Utilisation d'un composé de formule I selon l'une ou plusieurs des revendications 1 à 3 et/ou ses sels physiologiquement acceptables, dans laquelle R¹ représente CONR⁵R⁶, -CO-(alkyle en C₁ à C₁₀), -CO-aryle, -NH-CO- (alkyle en C₁ à C₁₀), -NH-CO-aryle, -N(CO-alkyle en C₁ à C₁₀)₂, -N(CO-aryle)₂, -NH-SO₂-alkyle en C₁ à C₁₀, -NH-SO₂-aryle, -N(SO₂-aryle)₂ ou -N(SO₂-alkyle en C₁ à C₁₀)₂, tous les radicaux alkyle pouvant être substitués par un ou plusieurs radicaux R⁴ identiques ou différents.

6. Utilisation d'un composé de formule I selon l'une ou plusieurs des revendications 1 à 5 et/ou ses sels physiologiquement acceptables pour la préparation d'un médicament destiné à la thérapie ou la prophylaxie de maladies cardiovasculaires, du dysfonctionnement endothélial, du dysfonctionnement diastolique, de l'athérosclérose, de l'hypertension, de l'angine de poitrine, de thromboses, de resténoses, de l'infarctus du myocarde, d'attaques, de l'insuffisance cardiaque, de l'hypertonie pulmonaire, du dysfonctionnement érectile, de l'asthme bronchique, de l'insuffisance rénale chronique, du diabète ou de la cirrhose du foie, ou à l'amélioration d'une aptitude limitée à l'apprentissage ou de la faculté de mémoire.

7. Composé de formule Ik 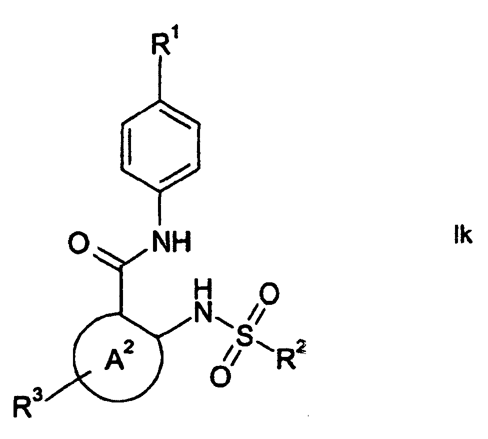 dans laquelle
le cycle A², qui comprend les atomes de carbone qui portent les groupes CO-NH- et NH-SO₂R², représente un cycle benzène ;
R¹ représente un substituant de la série CO-(alkyle en C₁ à C₁₀), CO-aryle, CO-NR⁵R⁶, -NH-CO-(alkyle en C₁ à C₁₀), -NH-CO-aryle, -N (CO-alkyle en C₁ à C₁₀)₂, -N(CO-aryle)₂, -NH-SO₂-alkyle en C₁ à C₁₀, -NH-SO₂-aryle, -N(SO₂-alkyle en C₁ à C₁₀)₂ et -N(SO₂-aryle)₂, où tous les radicaux alkyle peuvent être substitués par un ou plusieurs radicaux R⁴ identiques ou différents ;
R² représente phényle, qui peut être substitué par un ou plusieurs substituants identiques ou différents de la série halogène, CF₃, alkyle en C₁ à C₃, cyano, nitro et alcoxy en C₁ à C₃ ;
R³ représente un ou plusieurs substituants identiques ou différents de la série hydrogène et halogène ;
R⁴ représente fluor, OH, -O-alkyle en C₁ à C₁₀, -O-(alkyle en C₁ à C₇)-R⁷, -O-aryle, SH, -S-alkyle en C₁ à C₁₀, -S-(alkyle en C₁ à C₇)-R⁷, -S-aryle, -P(O)(O-alkyle en C₁ à C₅)₂, -P(O)(OH)₂, CN, NR⁸R⁹, CO-NH₂, CO-NH-(alkyle en C₁ à C₃), CO-N(alkyle en C₁ à C₃)₂, COOH, CO-O-(alkyle en C₁ à C₅), hétérocyclyle ou oxo;
R⁵ représente hydrogène, alkyle en C₁ à C₁₀, qui peut être substitué par un ou plusieurs substituants R⁴ identiques ou différents et/ou par aryle, aryle, hétérocyclyle, CO-(alkyle en C₁ à C₁₀), CO-aryle, CO-hétérocyclyle, SO₂-alkyle en C₁ à C₁₀, SO₂-aryle ou SO₂-hétérocyclyle ;
R⁶ présente, indépendamment de R⁵, une des significations indiquées pour R⁵, ou R⁵ et R⁶ représentent ensemble avec l'atome d' azote, auquel ils sont liés, un cycle à 5 jusqu'à 8 chaînons, saturé ou partiellement insaturé, qui peut contenir, outre l'atome d'azote qui porte les groupes R⁵ et R⁶, encore un ou plusieurs autres hétéroatomes de cycle de la série N, 0 et S et qui peut être substitué par un ou plusieurs substituants identiques ou différents de la série fluor, alkyle en C₁ à C₅, hydroxyalkyle en C₁ à C₃, -(alkyle en C₁ à C₃)-O-(alkyle en C₁ à C₄), aryle, CF₃, OH, -O-alkyle en C₁ à C₇, -O-aryle, -O-(alkyle en C₂ à C₄)-O-(alkyle en C₁ à C₇), (alkylène en C₂ à C₃)dioxy, NR⁸R⁹, CN, CO-NH₂, CO-NH-(alkyle en C₁ à C₃), CO-N(alkyle en C₁ à C₃)₂, COOH, CO-O-(alkyle en C₁ à C₅), CHO, CO-(alkyle en C₁ à C₅), S(O)ₙ-alkyle en C₁ à C₄, S(O)ₙ-NH₂, S(O)ₙ-NH-alkyle en C₁ à C₃, S (O)ₙ-N (alkyle en C₁ à C₃)₂, oxo, -(CH₂)ₘ-NH₂, -(CH₂)ₘ-NH-(alkyle en C₁ à C₄) et -(CH₂)ₘ-N(alkyle en C₁ à C₄)₂, où, dans le substituant -(CH₂)ₘ-N(alkyle en C₁ à C₄)₂, les deux groupes alkyle peuvent être liés par une simple liaison et forment alors avec l'atome d'azote qui les porte un cycle à 5 jusqu'à 7 chaînons, qui peut encore contenir, outre l'atome d'azote et les atomes de carbone, un atome d'oxygène, de soufre ou un groupe NR⁵ supplémentaire comme chaînon du cycle ;
R⁷ représente OH, -O-alkyle en C₁ à C₇, NH₂, -NH-alkyle en C₁ à C₄ ou -N(alkyle en C₁ à C₄)₂, où, dans le substituant N(alkyle en C₁ à C₄)₂, les deux groupes alkyle peuvent être liés par une simple liaison et forment alors avec l'atome d'azote qui les porte un cycle à 5 jusqu'à 7 chaînons, qui peut encore contenir, outre l'atome d'azote et les atomes de carbone, un atome d'oxygène, de soufre ou un groupe NR⁵ supplémentaire comme chaînon de cycle ;
R⁸ représente hydrogène ou alkyle en C₁ à C₇, qui peut être substitué par un ou plusieurs substituants identiques ou différents de la. série OH, -O-alkyle en C₁ à C₅, NH₂, -NH-alkyle en C₁ à C₄ et -N (alkyle en C₁ à C₄)₂, où, dans le substituant N(alkyle en C₁ à C₄)₂, les deux groupes alkyle peuvent être liés par une simple liaison et forment alors avec l'atome d'azote qui les porte un cycle à 5 jusqu'à 7 chaînons, qui peut encore contenir, outre l'atome d'azote et les atomes de carbone, un atome d'oxygène, de soufre ou un groupe NR⁵ supplémentaire comme chaînon de cycle ;
R⁹ présente, indépendamment de R⁸, une des significations de R⁸ ou CO-(alkyle en C₁ à C₄) ;
aryle représente phényle, naphtyle ou hétéroaryle, qui peuvent tous être substitués par un ou plusieurs substituants identiques ou différents de la série halogène, alkyle en C₁ à C₅, phényle, tolyle, CF₃, NO₂, OH, -O-alkyle en C₁ à C₅, -O-(alkyle en C₂ à C₄)-O-(alkyle en C₁ à C₃), (alkylène en C₁ à C₂)dioxy, NH₂, -NH-alkyle en C₁ à C₃, -N(alkyle en C₁ à C₃)₂, NH-CHO, -NH-CO- (alkyle en C₁ à C₅), CN, CO-NH₂, CO-NH-(alkyle en C₁ à C₃), CO-N(alkyle en C₁ à C₃)₂, COOH, CO-O-(alkyle en C₁ à C₅), hétérocyclyle, CHO, CO-(alkyle en C₁ à C₅), S(O)ₙ-alkyle en C₁ à C₄, S(O)ₙ-phényle, S(O)ₙ-tolyle, S(O)₂-NH₂, S(O)₂-NH-alkyle en C₁ à C₃ et S(O)₂-N(alkyle en C₁ à C₃)₂ ;
hétéroaryle représente le radical d'un hétérocycle monocyclique aromatique à 5 ou 6 chaînons ou un hétérocycle bicyclique aromatique à 8 jusqu'à 10 chaînons, qui contiennent à chaque fois un ou plusieurs hétéroatomes de cycle de la série N, O et S ;
hétérocyclyle représente le radical d'un hétérocycle monocyclique ou polycyclique, à 5 jusqu'à 11 chaînons, saturé ou partiellement insaturé, qui contient un ou plusieurs hétéroatomes de cycle de la série N, O et S et qui peut être substitué par un ou plusieurs substituants identiques ou différents de la série fluor, alkyle en C₁ à C₅, OH, -O-alkyle en C₁ à C₅, -O- (alkyle en C₂ à C₄)-O-(alkyle en C₁ à C₃), NH₂, -NH-alkyle en C₁ à C₃, -N (alkyle en C₁ à C₃)₂, CN, CO-NH₂, CO-NH-(alkyle en C₁ à C₃), CO-N(alkyle en C₁ à C₃)₂, COOH et CO-O-(alkyle en C₁ à C₅) ;
n vaut 0, 1 ou 2 ;
m vaut 2, 3 ou 4 ;
dans toutes ses formes stereoisomères et mélanges de celles-ci dans tous les rapports, et ses sels physiologiquement acceptables.

8. Composé de formule Ik selon la revendication 7 et/ou ses sels physiologiquement acceptables destinés à une utilisation comme médicament.

9. Préparation pharmaceutique, **caractérisée en ce qu'**elle contient un ou plusieurs composés de formule Ik selon la revendication 7 et/ou leurs sels physiologiquement acceptables, en plus de substances support et/ou d'additifs pharmaceutiquement irréprochables.
